(19) Europäisches Patentamt
European Patent Office
Office européen des brevets

(11) Numéro de publication: **0 258 096 B1**

(12) # FASCICULE DE BREVET EUROPEEN

(45) Date de publication de fascicule du brevet: **09.09.92**

(21) Numéro de dépôt: **87401734.6**

(22) Date de dépôt: **24.07.87**

(51) Int. Cl.5: **C07D 295/125**, C07C 233/77, C07D 311/96, C07C 215/28, C07D 295/084, A61K 31/165, A61K 31/395

(54) **Dérivés de l'indane, leur procédé de préparation, leur application à titre de médicaments, les compositions pharmaceutiques les renfermant et les intermédiaires obtenus.**

(30) Priorité: **21.08.86 FR 8611927**
**03.07.87 FR 8709450**

(43) Date de publication de la demande:
**02.03.88 Bulletin 88/09**

(45) Mention de la délivrance du brevet:
**09.09.92 Bulletin 92/37**

(84) Etats contractants désignés:
**AT BE CH DE ES GB GR IT LI LU NL SE**

(56) Documents cités:
**EP-A- 272 982**
**EP-A- 0 005 821**
**EP-A- 0 260 555**
**GB-A- 2 096 607**

(73) Titulaire: **ROUSSEL-UCLAF**
**35, Boulevard des Invalides**
**F-75007 Paris(FR)**

(72) Inventeur: **Clemence, François**
**2, rue Turgot**
**F-75009 Paris(FR)**
Inventeur: **Le Martret, Odile**
**42, Avenue de versailles**
**F-75016 Paris(FR)**
Inventeur: **Delevallee, Françoise**
**48-50, Avenue de La Dame Blanche**
**F-94120 Fontenay-sous-Bois(FR)**
Inventeur: **Fortin, Michel**
**12, Passage Cottin**
**F-75018 Paris(FR)**

(74) Mandataire: **Bourgouin, André et al**
**Département des Brevets ROUSSEL UCLAF**
**B.P. no 9**
**F-93230 Romainville(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services

## Description

L'invention concerne de nouveaux dérivés de l'indane, leur procédé de préparation, les nouveaux intermédiaires ainsi obtenus, leur application à titre de médicaments et les compositions pharmaceutiques les renfermant.

L'invention a pour objet les composés de formule (I) :

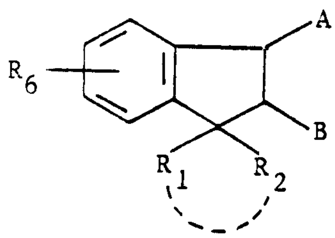

(I)

dans laquelle $R_6$ représente un atome d'hydrogène, un atome d'halogène, un radical alkyle ou alcoxy renfermant de 1 à 5 atomes de carbone et dans laquelle $R_1$ et $R_2$, identiques ou différents, représentent un atome d'hydrogène ou un radical alkyle renfermant de 1 à 5 atomes de carbone ou $R_1$ et $R_2$ forment ensemble avec l'atome de carbone auquel ils sont liés un cycle cycloalkyle renfermant entre 3 et 6 atomes de carbone et pouvant contenir éventuellement un hétéroatome tel qu'un atome d'oxygène ou de soufre ou d'azote et dans laquelle A et B sont tels que l'un des substituants A ou B est choisi dans

- le groupe de formule :

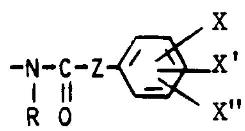

R étant un atome d'hydrogène ou un radical alkyle renfermant de 1 à 5 dans lequel $R_3$ et $R_4$, identiques ou différents, représentent un atome d'hydrogène ou un radial alkyle renfermant de 1 à 5 atomes de carbone ou $R_3$ et $R_4$ forment ensemble avec l'atome d'azote auquel ils sont liés un hétérocycle à 5 ou 6 chaînons comportant éventuellement un autre hétéroatome tel que l'oxygène, l'azote ou le soufre, l'atome d'azote étant éventuellement substitué par un radical alkyle renfermant de 1 à 4 atomes de carbone étant entendu que $R_6$ ne représente pas un radical méthoxy quand $R_1$ et $R_2$ représentent un atome d'hydrogène, B représente un radical pyrrolidinyle et A représente le radical 2,3-dichloro N-méthyl benzèneacétamide, lesdits composés de formule (I) pouvant être dans toutes les formes atomes de carbone Z une chaîne alkylène linéaire $-(CH_2)n-$, n étant un nombre entier pouvant varier entre 0 et 5 ou une chaîne alkylène ramifiée renfermant de 2 à 8 atomes de carbone ou un groupement $-CH_2-O-$, X, X' et X", identiques ou différents, représentent un atome d'hydrogène, un radical alkyle ou alkoxy renfermant de 1 à 4 atomes de carbone, un atome d'halogène, un radial hydroxyle, trifluorométhyle, nitro, amino, monoalkyl ou dialkylamido ou sulfoanimo et l'autre des substituants A ou B est choisi dans

- Le groupe de formule :

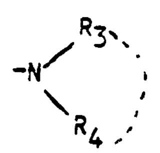

dans lequel $R_3$ et $R_4$, identiques ou différents, représentent un atome d'hydrogène ou un radical alkyle renfermant de 1 à 5 atomes de carbone ou $R_3$ et $R_4$ forment ensemble avec l'atome d'azote auquel ils sont liés un hétérocycle à 5 ou 6 chaînons comportant éventuellement un autre hétéroatome tel que l'oxygène, l'azote ou le soufren l'atome d'azote étant éventuellement substitué par un radical alkyle renfermant de 1 à 4 atomes de carbone étant entendu que $R_6$ ne représente pas un

radical méthoxy quand $R_1$ et $R_2$ représentent un atome d'hydrogène, B représente un radical pyrrolidinyle et A représente le radical 2,3-dichloro N-méthyl benzèneacétamide, lesdits composés de formule (I) pouvant être dans toutes les formes énantiomères et diastéréisomères possibles et sous forme de sels d'addition avec les acides.

Lorsque $R_1$, $R_2$, $R_3$, $R_4$, $R_6$, R, X, X' et X" représentent un radical alkyle, il s'agit de préférence des radicaux méthyle, éthyle, n-propyle ou isopropyle mais ces substituants peuvent également représenter un radical n-butyle, isobutyle, n-pentyle.

Lorsque $R_1$ et $R_2$ fomrent ensemble un radical cycloalkyle, il s'agit d'un radical cyclopropyle, cyclobutyle, cyclopentyle, cyclohexyle.

Lorsque $R_1$ et $R_2$ forment ensemble un radical cycloalkyle contenant un atome d'oxygène, de soufre ou d'azote, il s'agit respectivement d'un radical tétrahydropyrannyle, tétrahydrothiapyrannyle, pipéridinyle.

Lorsque Z représene un groupe $-(CH_2)n-$, n est de préférence égal à 0 ou à 1.

Lorsque Z est une chaîne alkylène ramifiée, il s'agit de préférence d'une chaîne substituée par un ou plusieurs radicaux méthyle ou éthyle. Il s'agit par exemple de la chaîne 1,1-éthanediyl; méthyl-1 éthanediyl-1,2; méthyl-1 ou 2 propanediyl-1,2; éthyl-1 éthanediyl-1,2.

Lorsque $R_6$, X, X' et X" représentent un radical alkoxy, il s'agit de préférence d'un radical méthoxy ou éthoxy mais X,X' et X" peuvent également représenter un radical propoxy, isopropoxy, butoxy linéaire, secondaire ou tertiaire.

Lorsque $R_6$, X, X′ et X″ sont des atomes d'halogène, il s'agit de préférence de l'atome de chlore mais X, X′ et X″ peuvent également représenter un atome de fluor, de brome ou d'iode.

Dans les valeurs monoalkyl et dialkylamino de X, X′ et X″, les radicaux alkyles sont préférentiellement les radicaux méthyle ou éthyle.

$R_3$ et $R_4$ forment de préférence avec l'atome d'azote auquel ils sont liés un radical pyridinyle, pipérazinyle, méthyl pipérazinyle, éthyl pipérazinyle ou propyl pipérazinyle, pipéridinyle, morpholinyle, pyrrolidinyle.

Les sels d'addition avec les acides minéraux ou organiques peuvent être, par exemple, les sels formés avec les acides chlorhydrique, bromhydrique, nitrique, sulfurique, phosphorique, acétique, propionique, formique, benzoïque, maléique, fumarique, succinique, tartrique, citrique, oxalique, glyoxylique, aspartique, alcanesulfoniques tels que l'acide méthanesulfonique et arylsulfoniques, tels que l'acide benzène sulfonique.

L'invention a notamment pour objet les composés de formule (I), caractérisés en ce que les groupements A et B sont en configuration trans ainsi que leurs sels d'addition avec les acides et ceux caractérisés en ce que dans le groupe

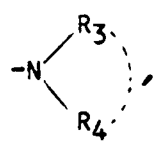

$R_3$ et $R_4$ représentent tous deux un radical méthyle ou forment avec l'atome d'azote auquel ils sont liés l'hétérocycle pyrrolidine, pipéridine, ou pipérazine éventuellement substitué par un radical alcoyle renfermant de 1 à 3 atomes de carbone, ainsi que leurs sels d'addition avec les acides.

L'invention a aussi particulièrement pour objet les composés de formule (I), caractérisés en ce que dans groupe

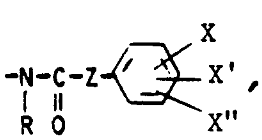

, R représente un atome d'hydrogène, un radical méthyle ou éthyle, Z un groupe $-(CH_2)_n$ dans lequel n est 0 ou 1,

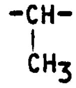

ou $-CH_2-O-$, ainsi que leurs sels d'addition avec les acides et ceux caractérisés en ce que $R_1$ et $R_2$ représentent tous deux un atome d'hydrogène, un radical méthyle ou forment ensemble avec l'atome de carbone auquel ils sont liés le cycle tétrahydropyranne ainsi que leurs sels d'addition avec les acides.

L'invention concerne aussi particulièrement les composés de formule (I), caractérisés en ce que X, X′ et X″, identiques ou différents, représentent un atome d'hydrogène, un radical méthyle ou éthyle, méthoxy ou éthoxy, nitro, sulfoamino, trifluorométhyle, un atome de chlore ainsi que leurs sels d'addition avec les acides et tout particulièrement :

- le [trans (±)] 3,4-dichloro N-[2,3-dihydro 2-(1-pyrrolidinyl) 1H-indèn-1-yl] N-méthyl benzène acétamide,
- le [trans (±)] N-[2,3-dihydro 2-(1-pyrrolidinyl) 1H-indèn-1-yl] N-méthyl 3-nitro benzène acétamide,
- le [trans (±)] N-[2,3-dihydro 2-(1-pyrrolidinyl) 1H-indèn-1-yl] N-méthyl 4-nitro benzène acétamide,
- le [trans (±)] N-[2,3-dihydro 2-(1-pyrrolidinyl) 1H-indèn-1-yl] N-méthyl (4-trifluorométhyl) benzène acétamide,
- le [trans (±)] 2-(3,4-dichlorophénoxy) N-[2,3-dihydro 2-(1-pyrrolidinyl) 1H-indèn-1-yl] N-méthyl acétamide,
- le [trans (±)] 3,4-dichloro N-[2,3-dihydro 2-(diméthylamino) 1H-indèn-1-yl] N-méthyl acétamide,
- le [trans (±)] N-[2,3-dihydro 2-(1-pyrrolidinyl) 1H-indèn-1-yl] N-méthyl 4-nitro benzène acétamide (isomère A) ainsi que leurs sels d'addition avec les acides.

L'invention a aussi pour objet un procédé de préparation des produits de formule (I) dont les groupements A et B sont en configuration trans, caractérisé en ce que l'on condense le produit de formule (II) :

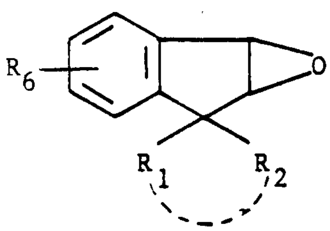

(II)

$R_6$, $R_1$ et $R_2$ ayant toutes les significations données précédemment,

- soit avec une amine de formule (III) :

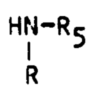

(III)

R ayant toutes les significations données précédemment et $R_5$ étant un groupement protecteur de la fonction amine et notamment un groupement benzyle, pour obtenir un produit de formule (IV) :

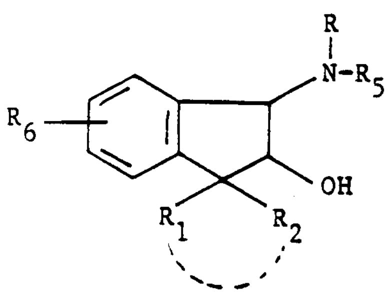

(IV)

dont on active la fonction hydroxyle et que l'on condense avec une amine de formule (V) :

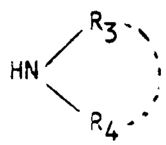

(V)

dans laquelle $R_3$ et $R_4$ ont les significations données précédemment pour obtenir un produit de formule (VI) :

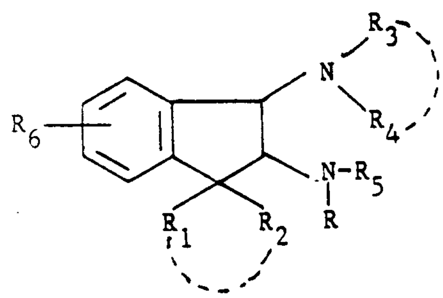

(VI)

dont on élimine le groupement protecteur $R_5$ de la fonction amine pour obtenir un produit de formule (VII) :

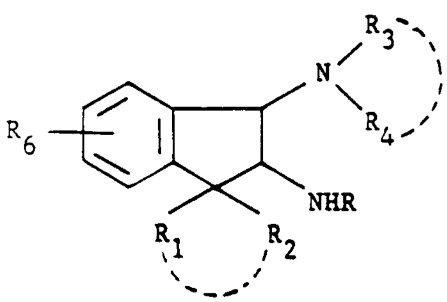

(VII)

que l'on traite avec un acide de formule (VIII) ou un dérivé fonctionnel de cet acide :

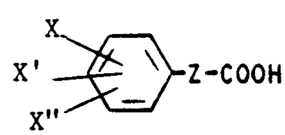

dans laquelle Z, X, X′ et X″ ont toutes les significations précédentes pour obtenir un produit de formule (I) dans laquelle A représente le groupe

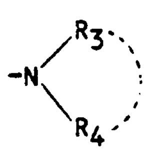 et B le groupe 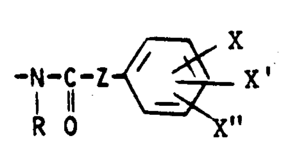 ,

- soit avec une amine de formule (V) :

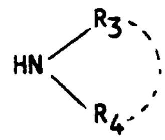

pour obtenir un produit de formule (IX) :

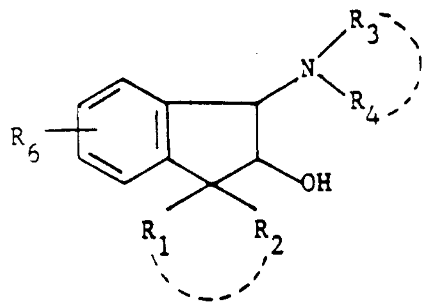

(IX)

dont on active la fonction hydroxyle et que l'on traite avec une amine de formule (X) :

$NH_2R$ (X)

dans laquelle R a la signification précédente pour obtenir un produit de formule (XI) :

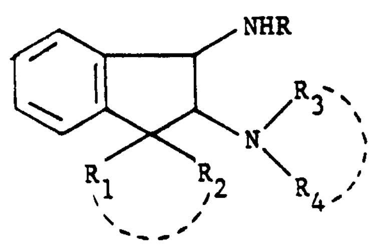

(XI)

que l'on condense avec un acide de formule (VIII) ou un dérivé fonctionnel de cet acide :

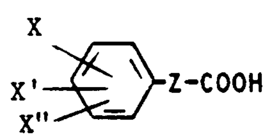

dans laquelle Z, X, X′ et X″ ont les significations précédentes pour obtenir un produit de formule (I) dans laquelle A représente le groupe

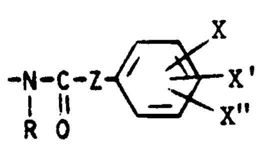 et B le groupe 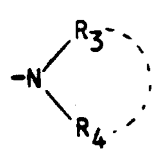 ,

lesdits composés de formule (I) pouvant être dédoublés pour obtenir les formes optiquement actives et que l'on traite, si désiré, avec un acide minéral ou organique, pour obtenir un sel.

Dans les conditions préférentielles du procédé de l'invention :

- Le chlorure de méthanesulfonyle est utilisé pour activer la fonction hydroxyle des produits de formule

(IV) et des produits de formule (IX).

- Dans les produits de formule (VI), le groupement de protection $R_5$ est un radical benzyle qui peut être éliminé par hydrogénation catalytique. Le catalyseur que l'on utilise est de préférence le palladium.
- L'activation de la fonction hydroxyle des composés de formule (VIII) pour réaliser la condensation avec le composé de formule (VII) ou (XI) s'effectue en présence de carbonyldiimidazole. On peut également activer les acides de formule (VIII) sous la forme d'un chlorure d'acide ou d'anhydride mixte.
- Les produits de formule (I) peuvent être dédoublés par les méthodes usuelles.

Les produits de formule (I) pour lesquels les groupements A et B sont en configuration cis peuvent être préparés notamment selon le schéma suivant :

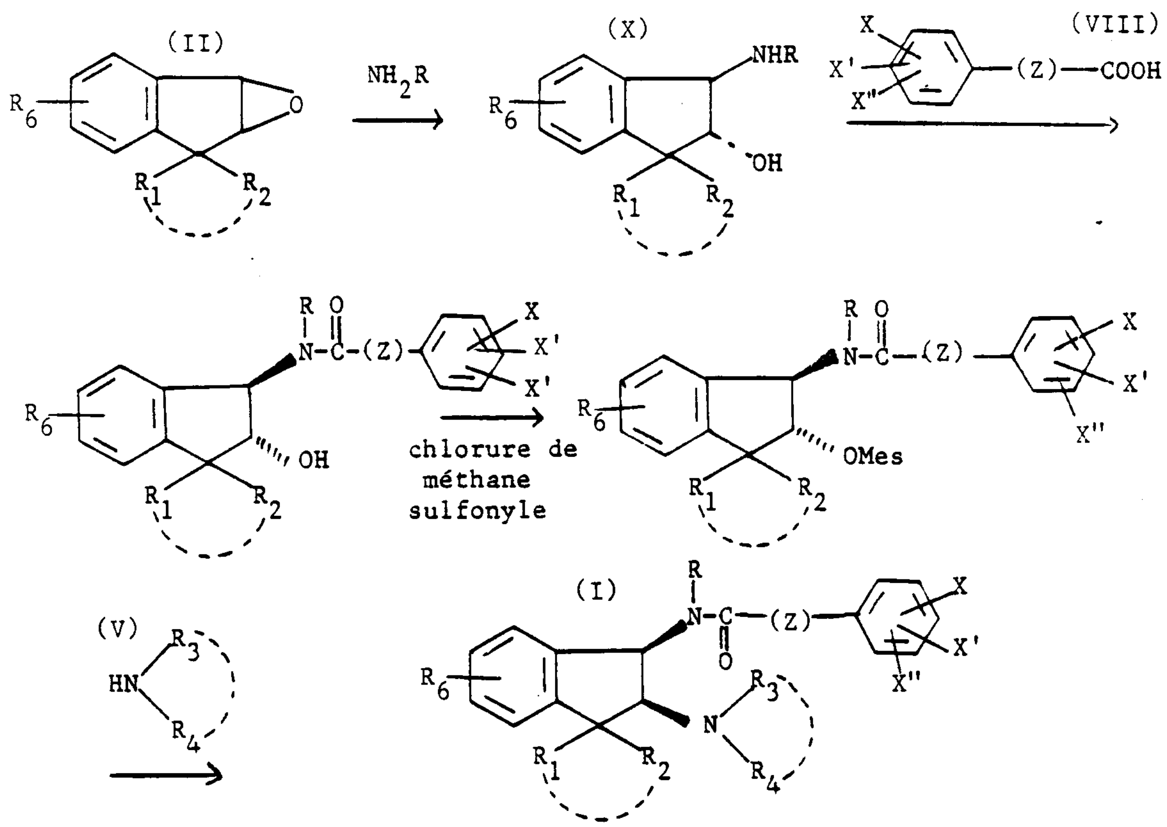


Les composés de formule (I) tels que définis ci-dessus ainsi que leurs sels d'addition avec les acides présentent d'intéressantes propriétés pharmacologiques. Ils présentent en particulier une forte affinité pour les récepteurs opiacés et notamment pour les récepteurs K et sont doués de propriétés analgésiques centrales.

Ils sont doués également de propriétés diurétiques, de propriétés anti-arythmiques, anti-ischémiques cérébrales et hypotensives.

Ces propriétés justifient leur application en thérapeutique et l'invention a également pour objet à titre de médicaments, les produits tels que définis par la formule (I) ci-dessus ainsi que par leurs sels d'addition avec les acides pharmaceutiquement acceptables.

La présente invention a tout particulièrement pour objet, à titre de médicament :

- le [trans (±)] 3,4-dichloro N-[2,3-dihydro 2-(1-pyrrolidinyl) 1H-indèn-1-yl] N-méthyl benzène acétamide,
- le [trans (±)] N-[2,3-dihydro 2-(1-pyrrolidinyl) 1H-indèn-1-yl] N-méthyl 3-nitro benzène acétamide,
- le [trans (±)] N-[2,3-dihydro 2-(1-pyrrolidinyl) 1H-indèn-1-yl] N-méthyl 4-nitro benzène acétamide,
- le [trans (±)] N-[2,3-dihydro 2-(1-pyrrolidinyl) 1H-indèn-1-yl] N-méthyl (4-trifluorométhyl) benzène acétamide,
- le [trans (±)] 2-(3,4-dichlorophénoxy) N-[2,3-dihydro 2-(1-pyrrolidinyl) 1H-indèn-1-yl] N-méthyl acétamide,
- le [trans (±)] 3,4-dichloro N-[2,3-dihydro 2-(diméthylamino) 1H-indèn-1-yl] N-méthyl acétamide,
- le [trans (±)] N-[2,3-dihydro 2-(1-pyrrolidinyl) 1H-indèn-1-yl] N-méthyl 4-nitro benzène acétamide

(isomère A) ainsi que leurs sels d'addition avec les acides.

Les médicaments, objet de l'invention, permettent notamment de soulager une douleur quelle qu'en soit l'origine, par exemple une douleur de nature musculaire, articulaire ou nerveuse.

Ils peuvent être aussi utilisés dans le traitement des douleurs dentaires, des migraines, du zona, dans le traitement des douleurs intenses, en particulier rebelles aux antalgiques périphériques, par exemple au cours du processus néoplasique, dans le traitement des pancréatites, coliques néphrétiques ou biliaires, dans le traitement des douleurs post-opératoires et post-traumatiques.

La posologie varie notamment en fonction de la voie d'administration, de l'affection traitée et du sujet en cause.

Par exemple, chez l'adulte, elle peut varier entre 20 et 400 mg de principe actif par jour, par voie orale et entre 5 et 100 mg par jour, par voie parentérale.

Les médicaments, objet de l'invention, trouvent aussi leur emploi dans le traitement des arythmies.

La dose usuelle, variable selon le dérivé utilisé, le sujet et l'affection en cause, peut être par exemple de 50 mg à 1 g par jour.

Par voie orale, le principe actif peut être administré à la dose quotidienne de 200 mg à 800 mg, par exemple pour le traitement des arythmies ventriculaires, supraventriculaires et jonctionnelles, soit environ de 3 mg à 12 mg par kilogramme de poids corporel.

Les médicaments, objet de l'invention peuvent aussi être utilisés dans le traitement des syndromes oedémateux, de l'insuffisance cardiaque, de certaines obésités, des cirrhoses, dans le traitement des oedèmes sévères et réfractaires, en particulier ceux de l'insuffisance cardiaque congestive et dans le traitement au long cours de l'hypertention artérielle.

La dose quotidienne de principe actif est variable. Elle peut être par exemple de 60 à 100 mg/jour par voie orale.

L'invention s'étend aux compositions pharmaceutiques renfermant comme principe actif les médicament définis ci-dessus.

Ces compositions pharmaceutiques peuvent être adminitrées par voie buccale, rectale, par voie parentérale ou par voie locale en application topique sur la peau et les muqueuses.

Ces compositions peuvent être solides ou liquides et se présenter sous les formes pharmaceutiques couramment utilisées en médecine humaine comme par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Par ailleurs, le produit de départ de formule (II) pour lequel $R_1$ et $R_2$ sont des atomes d'hydrogène ou des radicaux alcoyles renfermant de 1 à 5 atomes de carbone sont préparés par oxydation de l'indène correspondant.

Les autres produits de formule (II), et notamment ceux pour lesquels $R_1$ et $R_2$ forment un tétrahydropyranne, sont préparés selon le schéma suivant :

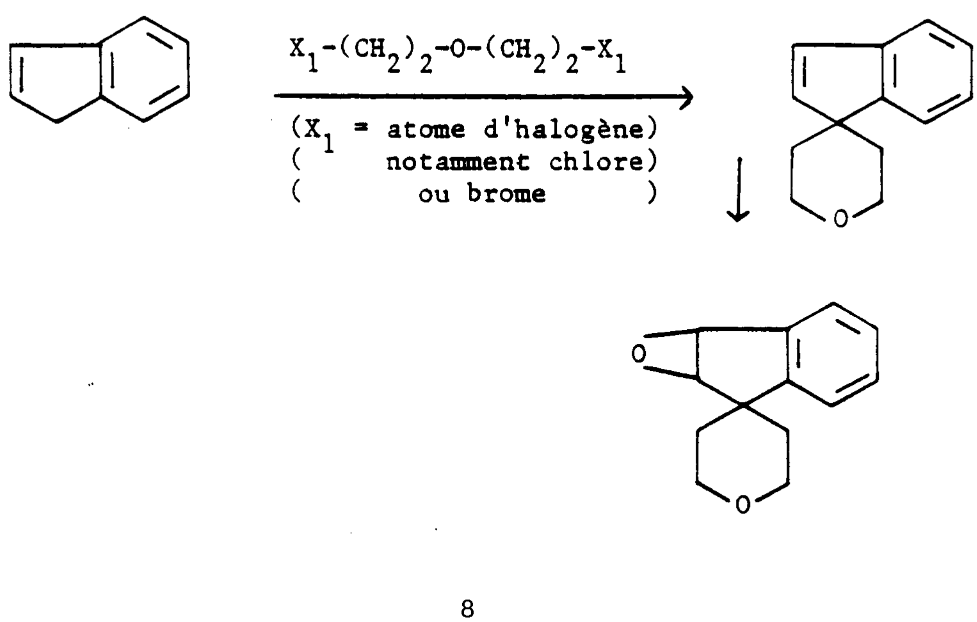

L'invention concerne aussi, à titre de produits industriels nouveaux, les produits de formule (IV), (VI), (VII), (IX), (XI) dans laquelle $R_6$ ne représente pas un atome d'hydrogène et R1 et R2 ne représentent pas un atome d'hydrogène ou un radical alkyle renfermant de 1 à 5 atomes de carbone.

Les exemples donnés ci-après illustrent l'invention sans toutefois la limiter.

**Exemple 1 : [Trans (+)] 3,4-dichloro N-[2,3-dihydro 2-(1-pyrrolidinyl) 1H-indèn-1-yl] N-méthyl benzène acétamide et son chlorhydrate.**

**Stade A** : [Trans (+)] 2,3-dihydro 1-(1-pyrrolidinyl) 1H-idèn-2-ol.

On ajoute lentement 10,8 cm3 de pyrrolidine dans 6,75 g de 2,3-époxy indane (décrit par Mousseron et coll., Bulletin de la Société Chimique de France, 1946, p. 629-630) dans 10,8 cm3 d'eau déminéralisée. La température augmente à 65¤C et on agite la solution 1 heure 30 minutes à cette température. On ajoute 20 cm3 d'eau déminéralisée en fin de réaction, distille l'excès de pyrrolidine sous pression réduite, obtient une phase huileuse et une phase aqueuse, sature au chlorure de sodium à 20¤C, ajoute 1 cm3 de lessive de soude à 32% et extrait à l'éther. On sèche, concentre par distillation sous pression réduite, purifie l'huile obtenue par chromatographie sur silice (éluant : acétate d'éthyle à 5% de triéthylamine) et obtient 8,81 g de produit attendu.

**Stade B :** [Trans (±)] 2,3-dihydro N-méthyl 2-(1-pyrrolidinyl) 1H-indèn-1-amine.

On refroidit à -20¤C un mélange renfermant 8,71 g de produit obtenu au stade A, 87 cm3 de chlorure de méthylène et 13,8 cm3 de triéthylamine et y introduit à cette température une solution renfermant 6,6 cm3 de chlorure de méthane sulfonyle et 8,7 cm3 de chlorure de méthylène. On agite 20 minutes à -20¤C, laisse revenir à 0¤C, lave à l'eau glacée, extrait les lavages par du chlorure de méthylène. On sèche les phases organiques, concentre à sec sous pression réduite et obtient 13,36 g d'une résine correspondant au Trans (±) méthane sulfonate de 2,3-dihydro 2-(1-pyrrolidinyl) 1H-indèn-2-ol. Le résidu est traité dans un autoclave par une solution aqueuse à 35-40% de monométhylamine. On chauffe à 80¤C durant 20 heures (pression stabilisée à 3 bars). On refroidit à 20¤C, reprend par 100 cm3 d'éther, sature au chlorure de sodium, décante, lave la phase organique à l'eau salée saturée. On sèche, traite au charbon actif, filtre, rince, concentre à sec sous pression réduite. On obtient 6,98 g d'une résine que l'on purifie par chromatographie sur silice (éluant : acétate d'éthyle-méthanol-triéthylamine 85-10-5). On obtient 3,71 g de produit attendu.

**Stade C :** [Trans (±)] 3,4-dichloro N-[2,3-dihydro 2-(1-pyrrolidinyl) 1H-indèn-1-yl] N-méthyl benzène acétamide et son chlorhydrate.

On agite pendant 1 heure 2,66 g d'acide 3,4-dichlorophénylacétique, 2,11 g de carbonyldiimidazole et 20 cm3 de tétrahydrofuranne puis ajoute lentement une solution de 2,16 g de produit obtenu au stade B dans 5 cm3 de tétrahydrofuranne. On agite pendant 3 heures 30 minutes, distille le tétrahydrofuranne sous pression réduite, reprend le résidu par 100 cm3 d'éther, lave par une solution saturée de bicarbonatê de sodium puis par de l'eau salée saturée, sèche, concentre à sec sous pressioon réduite et obtient 4,68 g de produit attendu sous forme de base.

Préparation du chlorhydrate.

On dissout à 50¤C le produit obtenu précédemment dans 10 cm3 d'éthanol 99, ajoute à chaud 3 cm3 d'une solution éthanolique d'acide chlorhydrique anhydre (titre = 5,75 N). On filtre aussitôt à chaud, rince à l'éthanol à 50¤C, amorce la cristallisation à 30¤C, laisse cristalliser au repos à 20¤C durant 2 heures. On essore, rince à l'éthanol et à l'éther, séche sous pression réduite et obtient 3,815 g du chlorhydrate attendu. F = 242¤C.

**Exemple 2 : [Trans (±)] 3,4-dichloro N-[2,3-dihydro 1-(1-pyrrolidinyl) 1H-indèn-2-yl] N-méthyl benzène acétamide et son fumarate.**

**Stade A :** [Trans (±)] 2,3-dihydro 1-[méthyl (phénylméthyl) amino] 1H-indèn-2-ol.

On agite à 95¤C pendant 1 heure 10,06 g de 2,3-époxy indane, 50 cm3 d'eau déminéralisée et 15 cm3

de N-méthyl benzylamine. On refroidit à 20¤C, ajoute 50 g de glace, filtre à 0¤C, + 5¤C la gomme obtenue, rince à l'eau, redissout par 250 cm3 d'acétate d'éthyle. On extrait successivement par 70 cm3, 50 cm3 et 30 cm3 d'acide chlorhydrique 2 N, lave les phases aqueuses par de l'acétate d'éthyle, ajoute 150 cm3 d'acétate d'éthyle aux extractions chlorhydriques, amène le pH à 9-10 par 25 g de bicarbonate de sodium, agite, puis ajoute 0,5 cm3 de lessive de soude. On décante, réextrait à l'acétate d'éthyle, lave à l'eau salée, sèche, traite au charbon actif, rince, concentre à sec sous pression réduite. On obtient 17,29 g de résine que l'on reprend par 60 cm3 de n-hexane, amorce la cristallisation à 20¤C, triture, essore, rince au n-hexane, sèche et obtient 15,95 g de produit attendu fondant à 64¤C.

**Stade B :** [Trans (±)] 1-[2,3-dihydro 2-[méthyl (phénylméthyl) amino] 1H-indèn-1-yl] pyrrolidine.

a) On refroidit à -20¤C une solution renfermant 13,95 g de produit obtenu au stade A, 98 cm3 de chlorure de méthylène et 23 cm3 de triéthylamine et introduit une solution de 8,5 cm3 de chlorure de méthane sulfonyle et de 21 cm3 de chlorure de méthylène. On agite 30 minutes à -20¤C, ramène à 0¤C en 5 minutes, lave à l'eau glacée, sèche, concentre à sec sous pression réduite et obtient 20,48 g d'une huile correspondant au méthane sulfonate de [trans (±)] 2,3-dihydro 1-[méthyl (phénylméthyl) amino] 1H-indén-2-ol.

b) On agite à 85¤C, 20,48 g de produit précédent dans 60 cm3 de pyrrolidine et 60 cm3 d'eau déminéralisée. On agite l'émulsion à 65¤C pendant 1 heure 30 minutes, distille l'excès de pyrrolidine sous pression réduite, reprend le résidu par un mélange eau-glace. On filtre la gomme obtenue, la rince à l'eau, la dissout avec de l'éther, décante l'eau, sèche la solution éthérée et concentre à sec sous pression réduite. On obtient 15,39 g d'une résine.

**Stade C :** [Trans (±)] 1-[2,3-dihydro 2-(méthylamino) 1H-indèn-1-yl] pyrrolidine.

On mélange 15,39 g de produit obtenu au stade B, 230 cm3 de méthanol, 15,5 cm3 d'acide chlorhydrique, 9,25 g de palladium à 10% sur charbon actif. On met 2 heures 30 minutes en hydrogénation sous agitation et 1800 mbars de pression d'hydrogène. On filtre le catalyseur et le charbon actif. On rince au méthanol, concentre le filtrat sous pression réduite et obtient une huile. On ajoute de l'éther, agite, alcalinise par 15 cm3 de lessive de soude à 32% en maintenant à 20¤C, agite, décante, réextrait la phase aqueuse à l'éther, sèche les phases organiques et concentre à sec sous pression réduite. On obtient 9,85 g de produit attendu.

**Stade D :** [Trans (±)] 3,4-dichloro N-[2,3-dihydro 1-(1-pyrrolidinyl) 1H-indèn-2-yl] N-méthyl benzène acétamide et son fumarate.

On agite 1 heure à 20-25¤C, 2,66 g d'acide 3,4-dichlorophénylacétique, 2,10 g de carbonyldiimidazole et 25 cm3 de tétrahydrofuranne puis ajoute 2,16 g de produit obtenu au stade B dans 10 cm3 de tétrahydrofuranne, agite 3 heures 30 minutes à 20-25¤C, distille le tétrahydrofuranne sous pression réduite. On reprend le résidu à l'éther, lave par une solution saturée de bicarbonate de sodium puis à l'eau saturée au chlorure de sodium. On sèche, concentre à sec sous pression réduite. On obtient 5,048 g de résine fluide correspondant au produit attendu brut sous forme de base.

Préparation du fumarate.

On dissout la base brute dans 50 cm3 d'éthanol 99, filtre, rince à l'éthanol, ajoute 1,25 g d'acide fumarique, chauffe pour le dissoudre. Le sel cristallise en refroidissant, on essore, rince à l'éthanol 99 et à l'éther, sèche à 65¤C sous pression réduite et obtient 4,908 g de produit que l'on recristallise d'abord dans l'isopropanol à 5% d'eau, puis dans l'éthanol 99 et obtient 4,038 g de produit attendu. F = 140¤C.

**Exemple 3 : [Trans (±)] 3,4-dichloro N-[2,2′,3,3′,5′,6′-hexahydro 2-(1-pyrrolidinyl) spiro [1H-indèn-1,4′-(4H)-pyran] 3-yl] N-méthyl benzène acétamide et son chlorhydrate.**

**Stade A :** 2,3,5,6-tétrahydro spiro pyran 4-(4H)-1'-indène.

On ajoute 250 cm3 de tétrahydrofuranne et 250 cm3 d'hexaméthylphosphorotriamide dans 53 g d'hydrure de sodium à 50% dans l'huile préalablement lavé à l'éther de pétrole puis introduit, sans dépasser 0¤C, 58 g d'indène dans 50 cm3 de tétrahydrofuranne. On laisse remonter la température à

15¤C et ajoute en 30 minutes à 16¤C ± 1¤ en agitant 71,5 g de béta-chloroéthyléther dans 50 cm3 de tétrahydrofuranne, agite encore 1 heure 30 minutes puis verse prudemment dans 1 litre d'acide chlorhydrique 2 N contenant environ 500 g de glace. On extrait à l'éther isopropylique, lave à l'eau. On sèche, agite avec du noir animal et de l'alumine, essore, concentre à sec sous pression réduite et obtient 100 g d'huile que l'on reprend par 200 cm3 d'éther de pétrole, amorce la cristallisation, glace 3 heures, essore, lave par de l'éther de pétrole glacé et obtient 55 g de produit. On amène les liqueurs mères à sec, reprend par 40 cm3 de chlorure de méthylène, chromatographie sur silice et obtient 25 g de produit que l'on recristallise dans l'éther de pétrole pour obtenir 14 g de produit attendu. F = 83¤C.

**Stade B :** 2′,3′-époxy 2,3,5,6-tétrahydro spiro [pyran 4-(4H)-1′indane].

On ajoute à 15¤C, 6,3 g d'acide métachloroperbenzoïque dans 5,58 g de produit obtenu au stade A dans 60 cm3 de chlorure de méthylène. On refroidit un peu pour ne pas dépasser 30¤C, après une demi-heure, l'acide m-chlorobenzoïque cristallise. On laisse 1 heure, verse dans un mélange d'eau-bicarbonate de sodium, extrait au chlorure de méthylène, lave avec une solution de bicarbonate de sodium, sèche, concentre à sec. Le résidu obtenu est cristallisé dans le chlorure de méthylène et l'éther isopropylique. On essore, lave à l'éther isopropylique, sèche à 80¤C et obtient 4,23 g de produit attendu. F = 157¤C.

**Stade C :** Chlorhydrate de [trans (±)] 2,2′,3,3′,5′,6′-hexahydro 3-(1-pyrrolidinyl) spiro [1H-indèn-1,4′-(4H)-pyran]-2-ol.

On chauffe à 60-70¤C, 11,66 g de produit obtenu au stade B dans 10 cm3 d'eau déminéralisée et 12,6 cm3 de pyrrolidine puis à 50¤C en agitant pendant 15 minutes, refroidit à 30¤C, dilue par 100 cm3 d'eau glacée, filtre, rince la gomme obtenue, rince à l'eau. On reprend la gomme par de l'éther, décante l'eau, sèche, concentre à sec sous pression réduite et obtient 12,8 g de produit attendu. On extrait les eaux mères aqueuses à l'éther, sèche, concentre à sec sous pression réduite et récupère 3,63 g de produit attendu.

Préparation du chlorhydrate.

On dissout les 16,43 g de produit obtenu dans 15 cm3 d'éthanol puis ajoute 11 cm3 d'une solution éthanolique d'acide chlorhydrique (5,75 N). Le chlorhydrate cristallise et on dilue lentement sous agitation la suspension par 55 cm3 d'éther, on essore, rince par un mélange éthanol-éther puis par de l'éther, sèche sous pression réduite à 60-65¤C et obtient 13,65 g de produit attendu. F = 183¤C.

**Stade D :** Méthane sulfonate de [trans (±)] 2,2′,3,3′,5′,6′-hexahydro 3-(1-pyrrolidinyl) spiro [1H-indèn-1,4′-(4H)-pyran] 2-ol.

On introduit 9,25 cm3 de triéthylamine dans une solution de 6,19 g de produit obtenu au stade C dans 40 cm3 de chlorure de méthylène. On refroidit à -15¤C et ajoute une solution de 3,1 cm3 de chlorure de méthane sulfonyle (0,04 M) dans 20 cm3 de chlorure de méthylène, agite 30 minutes à -15¤C ± 2¤, ramène à 0¤C la température, verse sur 100 cm3 d'eau à +10¤C, décante, extrait au chlorure de méthylène, lave à l'eau, sèche, ajoute de la silice, agite 5 minutes, filtre, rince, concentre à sec sous pression réduite et obtient 7,45 g d'une huile. On la dissout dans 30 cm3 d'éther à 20¤C, amorce la cristallisation, essore, rince à l'éther, sèche sous pression réduite et obtient 5,15 g de produit attendu. F = 136-137¤C.

**Stade E :** Dichlorhydrate de [trans (±)] 2,2′,3,3′,5′,6′-hexahydro N-méthyl 2-(1-pyrrolidinyl) spiro [1H-indèn-1,4′-(4H)-pyran]-3-amine.

On agite à 70¤C pendant 18 heures 30 minutes dans un autoclave 4,65 g de produit obtenu au stade D, 9,3 cm3 d'une solution aqueuse de méthylamine à 35-40%. La pression se stabilise à 1,2-1,4 bar. On refroidit, reprend le mélange obtenu par 20 cm3 d'eau salée saturée et 100 cm3 d'éther. On agite à 20¤C pour dissoudre la gomme, décante, extrait à l'éther, lave à l'eau salée, sèche, concentre à sec sous pression réduite. L'huile épaisse obtenue est dissoute dans 10 cm3 de n-hexane à 40¤C. On amorce la cristallisation, essore à 0¤, +5¤C, rince au n-hexane, sèche sous pression réduite et obtient 3,26 g de produit attendu. F = 88¤C.

Préparation du chlorhydrate.

On met 2,18 g de base en suspension dans 7 cm3 d'éthanol, ajoute 3,5 cm3 d'éthanol renfermant de l'acide chlorhydrique (5,75 N) anhydre à 20¤C, filtre, ajoute lentement 10 cm3 d'éther au filtrat. On essore les cristaux obtenus, rince par un mélange éthanol-éther et par de l'éther, sèche sous pression réduite à 60-65¤C et obtient 2,74 g de produit attendu. F = 270¤C.

**Stade F :** [Trans (±)] 3,4-dichloro N-[2,2′ 3,3′,5′,6′-hexahydro 2-(1-pyrrolidinyl) spiro [1H-indèn-1,4′-(4H)-pyran] 3-yl] N-méthyl benzène acétamide et son chlorhydrate.

On opère comme au stade C de l'exemple 1 à partir de 789 mg d'acide 3,4-dichlorophénylacétique dans 10 cm3 de tétrahydrofuranne, de 624 mg de carbonyldiimidazole, de 1 g de produit obtenu au stade E (base). On obtient 1,443 g de produit attendu. F = 131¤C après purification par le n-hexane.

Préparation du chlorhydrate.

On dissout 0,5 g de base dans 3 cm3 d'éthanol au reflux, filtre à chaud, rince à l'éthanol bouillant, ajoute au filtrat 0,4 cm3 de solution éthanolique d'acide chlorhydrique anhydre (5,75 N). On essore, rince à l'éthanol 100 et à l'éther, sèche sous pression réduite à 60-65¤C et obtient 504 mg de produit attendu. F = 230¤C.

**Exemple 4 : (E) butènedioate de [trans (±)] 3,4-dichloro N-[2,2′,3,3′,5′,6′-hexahydro 3-(1-pyrrolidinyl) spiro [1H-indèn-1,4′-(4H)-pyran]-2-yl] N-méthyl benzène acétamide.**

**Stade A :** [Trans (±)] 2,2′,3,3′,5′,6′-hexahydro 3-[méthyl (phénylméthyl) amino] spiro [1H-indèn-1,4′-(4H)-pyran]-2-ol.

On opère comme au stade A de l′exemple 2 à partir de 10,1 g de 2,3′-époxy 2,3,5,6-tétrahydro spiro /pyran/ 4-(4H)-1′-indane dans 10 cm3 de méthylbenzylamine et 50 cm3 d'eau déminéralisée. On obtient 12,79 g de produit attendu utilisé tel quel pour la suite de la synthèse.

Préparation du chlorhydrate.

On dissout 4,40 g de base brute dans 15 cm3 d'éthanol 100 à 20¤C, filtre et ajoute 4 cm3 de solution éthanolique d'acide chlorhydrique (5,75 N) anhydre, dilue par 24 cm3 d'éther à 20¤C. On amorce la cristallisation à 20¤C, ajoute 24 cm3 d'éther, essore à 20¤C, rince par un mélange éthanol-éther puis par de l'éther, sèche sous pression réduite à 60-65¤C et obtient 4,2 g de produit attendu. F = 192¤C.

**Stade B :** Méthane sulfonate de [trans (±)] 2,2′,3,3′,5′,6′-hexahydro 3-[méthyl (phénylméthyl) amino] spiro [1H-indèn-1,4′-(4H)-pyran]-2-ol.

On opère comme en a) du stade B de l'exemple 2 à partir de 12,79 g de produit obtenu au stade A ci-dessus dans 100 cm3 de chlorure de méthylène, de 16,5 cm3 de triéthylamine, de 6,1 cm3 de chlorure de méthane sulfonyle dans 30 cm3 de chlorure de méthylamine. On obtient 16,07 g de produit attendu.

**Stade C :** [Trans (±)] 2,2′,3,3′,5′,6′-hexahydro N-méthyl N-(phényl méthyl) 3-(1-pyrrolidinyl) spiro [1H-indèn-1,4′-(4H)-pyran]-2-amine.

On opère comme en b) du stade B de l'exemple 2 à partir des 16,07 g de produit obtenu précédemment, de 37 cm3 de pyrrolidine et de 37 cm3 d'eau déminéralisée. On obtient 14,16 g de produit attendu utilisé tel quel pour la suite de la synthèse.

Préparation du chlorhydrate.

On dissout en tiédissant 2,60 g de base brute dans 6,5 cm3 d'isopropanol et ajoute à 20¤C, 4 cm3 d'acide chlorhydrique anhydre (4,4 N) dans l'isopropanol, filtre, rince à l'isopropanol, ajoute au filtrat 42 cm3 d'éther. On essore la gomme obtenue, rince par l'éther, sèche sous pression réduite à 62-65¤C et obtient 2,82 g de produit attendu. F = 150¤C.

**Stade D :** Dichlorhydrate de [trans (±)] 2,2′ 3,3′,5′,6′-hexahydro N-méthyl 3-(1-pyrrolidinyl) spiro [1H-indèn-1,4′-(4H)-pyran]-2-amine.

On opère comme au stade C de L'exemple 2 à partir de 12,35 g de base brute obtenue au stade C, de 247 cm3 de méthanol et de 17,3 cm3 d'acide chlorhydrique. On utilise 7,4 g de palladium à 10% sur charbon actif pour une hydrogénation à 24-26¤C sous 1800 mbars durant 1 heure environ. En fin de réaction, on filtre le charbon actif et le catalyseur sous azote, rince par du méthanol et concentre le filtrat sous pression réduite. On dissout le résidu à 20¤C dans 60 cm3 d'isopropanol, amorce la cristallisation à 20¤C, laisse 2 heures à température ambiante. On essore, rince par de l'isopropanol puis par de l'éther, sèche sous pression réduite à 65¤C et obtient 8,80 g de produit brut. On dissout 5,48 g de ce produit dans 20 cm3 de méthanol, filtre, rince par du méthanol, concentre sous pression réduite, reprend par de l'isopropanol, dissout en tiédissant. On filtre à chaud, rince par de l'isopropanol bouillant, amorce la cristallisation, essore, rince par de l'isopropanol et de l'éther, sèche sous pression réduite et obtient 5,16 g de produit attendu anhydre sous forme de dichlorhydrate. F = 235¤C du chlorhydrate hydraté.

**Stade E :** (E) butènedioate de [trans (±)] 3,4-dichloro N-[2,2′,3,3′,5′,6′-hexahydro 3-(1-pyrrolidinyl) spiro [1H-indèn-1,4′-(4H)-pyran]-2-yl] N-méthyl benzène acétamide.

On opère comme au stade D de l'exemple 2 à partir de 2,05 g d'acide 3,4-dichlorophénylacétique, de 30 cm3 de tétrahydrofuranne, de 1,62 g de carbonyldiimidazole, de 2,6 cm3 de triéthylamine et de 3,27 g de produit obtenu au stade précédent. On obtient 4,61 g de produit attendu. On en purifie 4,546 g par chromatographie sur silice (éluant : chlorure de méthylène-acétate d'éthyle 1-1). On élimine le solvant et obtient 3,71 g de produit attendu.

Obtention du fumarate.

On dissout 3,474 g de base purifiée dans 15 cm3 d'éthanol, ajoute 941 mg d'acide fumarique, chauffe pour obtenir une solution. On filtre à chaud, rince à l'éthanol bouillant. Le filtrat cristallise à chaud, on essore les cristaux à 20¤C, rince à l'éthanol et à l'éther, sèche sous pression réduite à 60-65¤C et obtient 3,631 g de fumarate attendu. F = 152¤C.

On opère comme au stade C de l'exemple 1 en utilisant au départ la [trans (±)] 2,3-dihydro N-méthyl 2-(1-pyrrolidinyl) 1H-indèn-1-amine obtenue au stade B de l'exemple 1 et l'acide correspondant et obtient les produits des exemples 5 à 14.

**Exemple 5 : [Trans (±)] N-[2,3-dihydro 2-(1-pyrrolidinyl) 1H-indèn-1-yl] N-méthyl 4-(trifluorométhyl) benzène acémtamide et son chlorhydrate.**

En utilisant 0,650 g de produit obtenu au stade B de l'exemple 1 et 0,796 g d'acide 4-trifluorométhyl phényl acétique, on obtient 0,673 g du chlorhydrate attendu. F = 245¤C.

| Analyse : $C_{23}H_{25}F_3N_2O$, HCl : 438,923 | | | | | |
|---|---|---|---|---|---|
| Calculé : | C% 62,94 | H% 5,97 | N% 6,38 | F% 12,99 | Cl% 8,08 |
| Trouvé : | 62,7 | 6,2 | 6,1 | 12,9 | 7,2 |

**Exemple 6 : [Trans (±)] N-[2,3-dihydro 2-(1-pyrrolidinyl) 1H-indèn-1-yl] N-méthyl 3-(trifluorométhyl) benzène acétamide et son chlorhydrate.**

En utilisant 0,432 g de produit obtenu au stade B de l'exemple 1 et 0,530 g d'acide m-trifluorométhyl phényl acétique, on obtient 0,786 g de base puis 0,606 g de chlorhydrate attentu. F # 211¤C (décomposition).

| Analyse : $C_{23}H_{25}F_3N_2O$, HCl : 438,923 | | | | | |
|---|---|---|---|---|---|
| Calculé :<br>Trouvé : | C% 62,94<br>62,8 | H% 5,97<br>6,1 | N% 6,38<br>6,3 | F% 12,99<br>13,0 | Cl% 8,08<br>8,2 |

**Exemple 7 : [Trans (±)] N-[2,3-dihydro 2-(1-pyrrolidinyl) 1H-indèn-1-yl] N-méthyl 2-(trìfluorométhyl) benzène acétamide et son chlorhydrate.**

En utilisant 0,432 g du produit obtenu au stade B de l'exemple 1 et 0,530 g d'acide O-trifluorométhyl phényl acétique, on obtient 0,945 g de base puis 0,751 g du chlorhydrate attendu. F # 260¤C (décomposition).

| Analyse : $C_{23}H_{25}F_3N_2O$, HCl : 438,923 | | | | | |
|---|---|---|---|---|---|
| Calculé :<br>Trouvé : | C% 62,94<br>62,6 | H% 5,97<br>6,0 | N% 6,38<br>6,1 | F% 12,99<br>13,3 | Cl% 8,08<br>8,4 |

**Exemple 8 : [Trans (±)] N-[2,3-dihydro 2-(1-pyrrolidinyl) 1H-indèn-1-yl] N-méthyl 4-nitro benzène acétamide et son chlorhydrate.**

En utilisant 1,73 g de produit obtenu au stade B de l'exemple 1 et 1,88 g d'acide para-nitrophényl acétique, on obtient 3,7 g de base puis 0,33 g de chlorhydrate attendu. F = 236¤C.

| Analyse : $C_{22}H_{25}N_3O_3$, HCl : 415,923 | | | | |
|---|---|---|---|---|
| Calculé :<br>Trouvé : | C% 63,53<br>63,4 | H% 6,30<br>6,2 | N% 10,10<br>9,9 | Cl% 8,53<br>8,6 |

**Exemple 9 : [Trans (±)] N-[2,3-dihydro 2-(1-pyrrolidinyl) 1H-indèn-1-yl] N-méthyl 3-nitro benzène acétamide et son chlorhydrate.**

En utilisant 0,432 g de produit obtenu au stade B de l'exemple 1 et 0,471 g d'acide m-nitrophényl acétique, on obtient 0,838 g de base puis 0,58 g de chlorhydrate attendu. F # 160¤C.

| Analyse : $C_{22}H_{25}N_3O_3$, HCl : 415,923 | | | | |
|---|---|---|---|---|
| Calculé :<br>Trouvé : | C% 63,53<br>63,6 | H% 6,30<br>6,4 | N% 10,10<br>10,1 | Cl% 8,53<br>8,5 |

**Exemple 10 : [Trans (±)] N-[2,3-dihydro 2-(1 pyrrolidinyl) 1H-indèn-1-yl] N-méthyl 2-nitro benzène acétamide et son chlorhydrate.**

En utilisant 0,432 g de produit obtenu au stade B de l'exemple 1 et 0,471 g d'acide O-nitrophényl acétique, on obtient 0,492 g du chlorhydrate attendu. F # 215¤C.

| Analyse : $C_{22}H_{25}N_3O_3$, HCl : 415,923 | | | | |
|---|---|---|---|---|
| Calculé :<br>Trouvé : | C% 63,53<br>63,6 | H% 6,30<br>6,4 | N% 10,10<br>10,1 | Cl% 8,53<br>8,5 |

**Exemple 11 : [Trans (±)] N-[2,3-dihydro 2-(1-pyrrolidinyl) 1H-indèn-1-yl] N-méthyl 3,4,5-triméthoxy**

**benzène acétamide et son chlorhydrate.**

En utilisant 0,432 g de produit obtenu au stade B de l'exemple 1 et 0,588 g d'acide 3,4,5-triméthoxyphényl acétique, on obtient 1,070 g de base puis 0,789 g de chlorhydrate attendu. F = 217¤C.

| Analyse : $C_{25}H_{32}N_2O_4$, HCl : 461,006 | | | | |
|---|---|---|---|---|
| Calculé :<br>Trouvé : | C% 65,13<br>65,0 | H% 7,21<br>7,3 | N% 6,08<br>5,9 | Cl% 7,69<br>7,4 |

**Exemple 12 : [Trans (±)] N-[2,3-dihydro 2-(1 pyrrolidinyl) 1H-indèn-1-yl] 4-N-diméthyl benzène acétamide et son chlorhydrate.**

En utilisant 0,650 g de produit obtenu au stade B de l'exemple 1 et 0,586 g d'acide para-tolyl acétique, on obtient 1,10 g de base puis 0,665 g du chlorhydrate attendu. F = 212¤C.

| Analyse : $C_{23}H_{28}N_2O$, HCl : 384,948 | | | | |
|---|---|---|---|---|
| Calculé :<br>Trouvé : | C% 71,76<br>71,8 | H% 7,59<br>7,6 | N% 7,28<br>7,1 | Cl% 9,21<br>9,3 |

**Exemple 13 : [Trans (±)] N-[2,3-dihydro 2-(1-pyrrolidinyl) 1H-indèn-1-yl] 3,4-diméthoxy N-méthyl benzène acétamide et son trans butène dioate.**

En utilisant 0,650 g de produit obtenu au stade B de l'exemple 1 et 0,765 g d'acide 3,4-diméthoxy phényl acétique, on obtient 1,14 g de base. A 1 g de ce produit, on ajoute 0,35 g d'acide fumarique dans 7 cm3 d'éthanol et obtient après filtration 0,886 g du sel attendu. F = 186¤C.

| Analyse : $C_{24}H_{30}N_2O_3$, $C_4H_4O_4$ 510,583 | | | |
|---|---|---|---|
| Calculé :<br>Trouvé : | C% 65,87<br>66,1 | H% 6,71<br>6,4 | N% 5,49<br>5,3 |

**Exemple 14 : [Trans (±)] 2-(3,4-dichlorophénoxy) [2,3-dihydro 2-(1-pyrrolidinyl) 1H-indèn-1-yl] N-méthyl acétamide et son chlorhydrate.**

En utilisant 0,432 g de produit obtenu au stade B de l'exemple 1 et 0,575 g d'acide 3,4-dichlorophénoxy acétique, on obtient 0,790 g de base puis 0,687 g de chlorhydrate attendu. F = 196¤C.

| Analyse : $C_{22}H_{24}Cl_2N_2O_2$, HCl : 455,815 | | | | |
|---|---|---|---|---|
| Calculé :<br>Trouvé : | C% 57,97<br>58,0 | H% 5,53<br>5,5 | N% 6,14<br>6,2 | Cl% 23,33<br>23,0 |

**Exemple 15 : [Trans (±)] 3,4-dichloro N-[2,3-dihydro 2-(1-pyrrolidinyl) 1H-indèn-1-yl] N-méthyl benzamide et son chlorhydrate.**

On ajoute 0,670 g de chlorure d'acide 3,4-dichlorobenzoïque dans l'éther à 0,650 g de produit obtenu au stade A de l'exemple 1 dans l'éther et agite 16 heures à température ambiante. On verse sur de l'eau glacée, sépare par décantation la phase éthérée, la lave avec une solution aqueuse saturée en bicarbonate de sodium puis à l'eau salée, extrait à l'éther, sèche et élimine les solvants sous pression réduite. On obtient 1,2 g de produit attendu sous forme de base. On dissout 1,1 g de la base dans 25 cm3 d'éthanol,

ajoute 0,8 cm3 d'une solution éthanolique de chlorure d'hydrogène (5,75 N) et recueille 1,05 g du chlorhydrate attendu. F = 260¤C.

| Analyse : $C_{21}H_{22}Cl_2N_2O$, HCl : 425,789 | | | | |
|---|---|---|---|---|
| Calculé : | C% 59,24 | H% 5,44 | N% 6,58 | Cl% 24,98 |
| Trouvé : | 59,0 | 5,5 | 6,6 | 24,7 |

**Exemple 16 : Trans N-[2,3-dihydro 2-(1-pyrrolidinyl) 1H-indèn-1-yl] N-méthyl 4-nitro benzène acétamide (isomère A) et son chlorhydrate.**

**Stade A :** Dédoublement de la trans (±) 2,3-dihydro N-méthyl 2-(1-pyrrolidinyl) 1H-indèn-1-amine.

. Isomère A :

On ajoute 12,1 g d'acide D (±) di-p-tolyl tartrique en solution dans 22,5 cm3 de méthanol dans une solution de 6,48 g de produit obtenu comme au stade B de l'exemple 1 dans 7,5 cm3 de méthanol et maintient en contact pendant 4 heures à +4¤C. On essore et recueille 5,293 g de sel isomère A après une nouvelle recristallisation dans le méthanol. F ≃ 170-178¤C.
$alpha_D$ = + 97,5¤ ± 5¤ (c = 0,26% $H_2O$).

On reprend le sel isomère A dans 40 cm3 d'eau et 150 cm3 d'éther et ajoute 40 cm3 de lessive de soude ; on sépare par décantation la phase aqueuse, sature en chlorure de sodium et extrait à l'éther. On sèche, élimine le solvant sous pression réduite et obtient 1,852 g de l'isomère A sous forme de base. F < 50¤C.
$alpha_D$ = - 10,5¤ ± 1¤ (c = 1% $CH_3OH$).

. Isomère B :

On concentre à sec le méthanol recueilli après filtration du sel isomère A ci-dessus, reprend le résidu dans l'eau, l'éther et la lessive de soude comme ci-dessus et obtient après extraction à l'éther et élimination du solvant 4,1 g de base que l'on dissout dans 6 cm3 de méthanol et ajoute une solution de 7,68 g d'acide L(-) di-p-tolyl tartrique dans 20 cm3 de méthanol. On amorce la cristallisation, abandonne 4 heures, essore et obtient après recristallisation dans le méthanol 3,208 g de sel isomère B. F ≃ 170¤C.
$alpha_D$ = -19,5¤ ± 2¤ (c = 0,5% DMF).

On reprend le sel isomère B dans l'eau et l'éther puis ajoute de la lessive de soude comme ci-dessus. Après extraction à l'éther et élimination du solvant sous pression réduite, on obtient 1,117 g de l'isomère B sous forme de base. F < 50¤C.
$alpha_D$ = + 10,5¤ ± 1¤ (c = 1% $CH_3OH$).

**Stade B :**

On opère comme au stade C de l'exemple 1 en utilisant au départ 0,649 g de l'isomère A sous forme de base préparé au stade précédent et 0,707 g d'acide p-nitrophényl acétique et obtient 1,361 g de produit attendu sous forme de base puis 0,957 g de chlorhydrate. F # 238¤C.
$alpha_D$ = + 85¤ ± 1,5¤(c = 1% $H_2O$).

| Analyse : $C_{22}H_{25}N_3O_3$, HCl : 415,923 | | | | |
|---|---|---|---|---|
| Calculé : | C% 63,53 | H% 6,30 | N% 10,10 | Cl% 8,53 |
| Trouvé : | 63,4 | 6,3 | 10,1 | 8,5 |

**Exemple 17 : N-[2,3-dihydro 2-(1-pyrrolidinyl) 1H-indèn-1-yl] N-méthyl 4-nitro benzène acétamide (isomère B) et son chlorhydrate.**

On opère comme au stade C de l'exemple 1 en utilisant au départ 0,649 g de l'isomère B sous forme

de base préparé à l'exemple 16 stade A et 0,707 g de l'acide p-nitrophényl acétique et obtient 1,369 g de produit attendu sous forme de base puis 0,982 g de chlorhydrate. F # 238¤C.
alpha$_D$ = - 80¤ ± 1,5¤ (c = 1% $H_2O$).

| Analyse : $C_{22}H_{25}N_3O_3$, HCl : 415,923 | | | | |
|---|---|---|---|---|
| Calculé :<br>Trouvé : | C% 63,53<br>63,5 | H% 6,30<br>6,3 | N% 10,10<br>10,2 | Cl% 8,53<br>8,7 |

**Exemple 18 : [Trans (±)] N-[2,3-dihydro 2-(1-pyrrolidinyl) 1H-indèn-1-yl] 2,4-dinitro N-méthyl benzène acétamide et son chlorhydrate.**

**Stade A :** Oxalate du [trans (±)] 2,3-dihydro N-méthyl 2-(1-pyrrolidinyl) 1H-indèn-1-amine.

On dissout 1,94 g du produit obtenu au stade B de l'exemple 1 dans 2 cm3 d'éthanol, ajoute à 60¤C 1,35 g d'acide oxalique, dihydraté, refroidit, essore et rince à l'éthanol puis à l'éther. On obtient 2,3 g de produit brut que l'on recristallise dans le méthanol à 5% d'eau. F = 215¤C.

| Analyse : | | | |
|---|---|---|---|
| Calculé :<br>Trouvé : | C% 58,11<br>58,0 | H% 6,60<br>6,7 | N% 7,97<br>7,7 |

**Stade B :** [Trans (±)] N-[2,3-dihydro 2-(1-pyrrolidinyl) 1H-indèn-1-yl] 2,4-dinitro N-méthyl benzène acétamide et son chlorhydrate.

On opère comme au stade C de l'exemple 1 au départ de 0,703 g de l'oxalate préparé au stade A ci-dessus et 0,588 g d'acide 2,4-dinitrophényl acétique. On obtient 0,81 g de produit brut que l'on chromatographie sur silice (éluant : acétate d'éthyle puis acétate d'éthyle à 5% de triéthylamine). On obtient 0,359 g de produit attendu sous forme de base puis 0,332 g de chlorhydrate. F > 260¤C (décomposition).

| Analyse : $C_{22}H_{24}N_4O_5$, HCl : 460,92 | | | | |
|---|---|---|---|---|
| Calculé :<br>Trouvé : | C% 57,32<br>57,4 | H% 5,47<br>5,5 | N% 12,16<br>12,0 | Cl% 7,69<br>8,0 |

**Exemple 19 : [Trans (±)] 3,5-bis-(trifluorométhyl) N-[2,3-dihydro 2-(1-pyrrolidinyl) 1H-indèn-1-yl] N-méthyl benzène acétamide et son chlorhydrate.**

On opère comme au stade C de l'exemple 1 en utilisant 0,432 g de produit préparé au stade B de l'exemple 1 et l'acide 3,5-bis-trifluorométhyl phényl acétique et obtient le chlorhydrate attentu avec un rendement de 68%. F # 226¤C (décomposition).

| Analyse : | | | | | |
|---|---|---|---|---|---|
| Calculé :<br>Trouvé : | C% 58,86<br>57,0 | H% 4,97<br>5,0 | N% 5,52<br>5,5 | F% 22,49<br>22,8 | Cl% 6,99<br>7,2 |

**Exemple 20 : [Trans (±)] N-[2,3-dihydro 2-(1-pyrrolidinyl) 1H-indèn-1-yl] N-méthyl 3,4,5-trìméthoxy benzamide et son chlorhydrate.**

On mélange à température ambiante 1 g de l'oxalate préparé au stade A de l'exemple 18 et 0,9 cm3 de triéthylamine dans le tétrahydrofuranne et ajoute 0,515 g de chlorure de l'acide 3,4,5-triméthoxy benzoique dans 5 cm3 de tétrahydrofuranne. On agite 16 heures, verse dans 30 cm3 d'eau glacée, décante, lave la phase organique avec une solution aqueuse de bicarbonate de soude puis à l'eau salée, extrait à l'éther, sèche et élimine les solvants sous pression réduite. On obtient 0,457 g de produit attendu sous forme de base F = 174¤C que l'on dissout dans 1 cm3 d'une solution éthanolique de chlorure d'hydrogène (≃ 1,68 N) et élimine les solvants sous pression réduite. On reprend le résidu dans 5 cm3 d'éther, amorce la cristallisation, essore et sèche à 80¤C sous pression réduite le chlorhydrate attendu. F # 210¤C.

| Analyse : $C_{24}H_{30}N_2O_4$, HCl : 446,979 | | | | |
|---|---|---|---|---|
| Calculé :<br>Trouvé : | C% 64,49<br>64,7 | H% 6,99<br>7,0 | N% 6,27<br>6,2 | Cl% 7,93<br>8,2 |

**Exemple 21 : [Trans(±)] 3-(aminosulfonyl) 4-chloro N-[2,3-dihydro 2-(1-pyrrolidinyl) 1H-indèn-1-yl] N-méthyl benzamide et son chlorhydrate.**

On opère comme à l'exemple 20 au départ de 0,907 g du produit obtenu au stade B de l'exemple 1 et du chlorure de l'acide 4-chloro 3-aminosulfonyl benzoïque. On obtient le chlorhydrate attendu avec un rendement de 66%. F > 260¤C.

| Analyse : | | | | | |
|---|---|---|---|---|---|
| Calculé :<br>Trouvé : | C% 53,62<br>53,4 | H% 5,35<br>5,6 | N% 8,93<br>8,6 | S% 6,82<br>6,7 | Cl% 15,07<br>14,8 |

Le chlorure de l'acide 4-chloro 3-aminosulfonyl benzoïque utilisé au départ de l'exemple a été préparé comme suit :

On porte au reflux sous agitation pendant 2 heures, 1 g d'acide 3-sulfonyl 4-chloro benzoïque dans 5 cm3 de chlorure de thionyle. On élimine sous pression réduite l'excès de chlorure de thionyle et obtient 1,098 g de produit attendu. F = 158¤C.

**Exemple 22 : [Trans (±)] 3,4-dichloro N-[2,3-dihydro 2-(1-pyrrolidinyl) 1H-indèn-1-yl] N,alpha-diméthyl benzène acétamide isomère A et isomère B et leurs chlorhydrates.**

On agite pendant 5 heures à température ambiante 2,30 g d'acide alpha-méthyl 3,4-dichlorophényl acétique, 1,73 g de produit obtenu au stade B de l'exemple 1, 0,08 g de diméthylamino pyridine et 2,55 g de dicyclohexylcarbodiimide dans du chlorure de méthylène. On filtre l'urée formée, concentre à sec le filtrat sous pression réduite, reprend le résidu dans l'éther, lave avec une solution aqueuse saturée en bicarbonate de sodium puis à l'eau salée, réextrait à l'éther, sèche et élimine les solvants sous pression réduite. On obtient 4 g de produit brut sous forme de base que l'on chromatographie sur silice (éluant : acétate d'éthyletriéthylamine 98-2) et recueille 1,05 g d'isomère A et 1,0 g d'isomère B.

. Isomère A : Préparation du chlorhydrate.

On ajoute 0,6 cm3 d'une solution éthanolique de chlorure d'hydrogène (5,75 N) à 0,90 g d'isomère A sous forme de base dans 8 cm3 d'éthanol. On recueille 0,7 g de chlorhydrate attendu après recristallisation dans l'isopropanol. F = 174¤C.

| Analyse : $C_{23}H_{26}Cl_2N_2O$, HCl : 453,839 | | | | |
|---|---|---|---|---|
| Calculé :<br>Trouvé : | C% 60,87<br>60,9 | H% 6,00<br>6,4 | N% 6,17<br>6,1 | Cl% 23,43<br>23,0 |

. Isomère B : Préparation du chlorhydrate.

On opère comme pour l'isomère A en partant de 0,90 g d'isomère B sous forme de base et obtient 0,55 g de chlorhydrate attendu. F = 176¤C.

**Exemple 23 : [Trans(±)] 3,4-dichloro N-[2,3-dihydro 2-(1-pipéridinyl) 1H-indèn-1-yl] N-méthyl benzène acétamide et son chlorhydrate.**

**Stade A :** [Trans (±)] 2,3-dihydro 1-pipéridinyl 1H-indèn-2-ol.

On ajoute à 50¤C en 5 minutes 50 cm3 de pipéridine et 50 cm3 d'eau dans 21,25 g de 2,3-époxy indane et maintient 2 heures sous agitation à cette température. On refroidit la solution à 20¤C, sature en chlorure de sodium, ajoute 5 cm3 de lessive de soude puis extrait à l'éther. On concentre à sec sous pression réduite, reprend le résidu dans 200 cm3 d'acétate d'éthyle à 40¤C, filtre, sèche et élimine les solvants sous pression réduite. On reprend le résidu dans l'éther isopropylique, filtre et amène à sec. On obtient 26,82 g de produit brut que l'on purifie par chromatographie sur silice (éluant : acétate d'éthyle à 1% de triéthylamine). F # 82¤C.

**Stade B :** [Trans (±)] 2,3-dihydro N-méthyl 2-pipéridinyl 1H-indèn-1-amine.

On refroidit à -20¤C une solution comprenant 4,34 g de produit obtenu au stade A, 3,7 cm3 de triéthylamine dans 30 cm3 de tétrahydrofuranne, ajoute en 7 minutes 1,9 cm3 de chlorure de méthane sulfonyle en solution dans 4 cm3 de tétrahydrofuranne, maintient sous agitation pendant 15 minutes puis laisse revenir à 0¤C. On ajoute 17 cm3 de méthylamine en solution éthanolique à 33%, agite en laissant revenir à température ambiante le mélange réactionnel. Après 25 heures d'agitation, on élimine le solvant sous pression réduite, reprend le résidu dans 10 cm3 d'eau salée, ajoute 5 cm3 de lessive de soude et extrait à l'acétate d'éthyle On concentre à sec et obtient 4,743 g de produit brut que l'on dissout dans 23 cm3 de solution éthanolique de chlorure d'hydrogène (1,68 N), amorce la cristallisation, essore, sèche à 70¤C et recueille 5,12 g de produit attendu. F > 260¤C.

| Analyse : | | | | |
|---|---|---|---|---|
| Calculé :<br>Trouvé : | C% 59,4<br>59,3 | H% 7,98<br>8,0 | N% 9,24<br>9,2 | Cl% 23,38<br>22,8 |

Stade C : [Trans (±)] 3,4-dichloro N-[2,3-dihydro 2-(1-pipéridinyl) 1H-indèn-1-yl] N-méthyl benzène acétamide et son chlorhydrate.

On agite pendant 1 heure 0,533 g d'acide 3,4-dichlorophényl acétique 0,422 g de carbonyldiimidazole dans 5 cm3 de tétrahydrofuranne, ajoute 0,6 cm3 de triéthylamine puis 0,606 g du produit obtenu au stade B ci-dessus. On agite 16 heures, élimine le solvant sous pression réduite, reprend le résidu dans 30 cm3 d'acétate d'éthyle, lave avec une solution aqueuse saturée de bicarbonate de sodium puis avec une solution saturée chlorure de sodium, sèche et concentre à sec sous pression réduite. On obtient 0,981 g de produit brut.

Préparation du chlorhydrate.

On dissout la base obtenue ci-dessus dans 5 cm3 d'éthanol, ajoute 1,5 cm3 de solution éthanolique de chlorure d'hydrogène (1,68 N) et amorce la cristallisation. On essore, rince à l'éthanol puis à l'éther, sèche à 80¤C sous pression réduite et recueille 0,656 g de produit attendu. F # 217¤C.

| Analyse : | | | | |
|---|---|---|---|---|
| Calculé :<br>Trouvé : | C% 60,87<br>60,7 | H% 6,00<br>6,0 | N% 6,17<br>6,0 | Cl% 23,43<br>23,3 |

**Exemple 24 : [Trans (±)] 3,4-dichloro N-[2,3-dihydro 2-(4-méthyl 1-pipérazinyl) 1H-indèn-1-yl] N-**

**méthyl benzène acétamide.**

**Stade A :** [Trans (±)] 2,3-dihydro 1-(4-méthyl 1-pipérazinyl) 1H-indèn-2-ol.

On opère comme à l'exemple 23 stade A à partir de 14,57 g de 2,3-époxy indant et 12,4 cm3 de N-méthyl pipérazine. On obtient 6,447 g de produit attendu. F = 132¤C.

**Stade B :** [Trans (±)] 2,3-dihydro N-méthyl 2-(4-méthyl 1-pipérazinyl) 1H-indèn-1-amine et son chlorhydrate.

On opère comme à l'exemple 23 stade B à partir de 4,64 g de produit obtenu au stade A ci-dessus et obtient 5,58 g de produit attendu sous forme de base puis 6,79 g de chlorhydrate. F > 260¤C.

| Analyse : | | | | |
|---|---|---|---|---|
| Calculé :<br>Trouvé : | C% 50,79<br>51,3 | H% 7,39<br>7,4 | N% 11,84<br>11,8 | Cl% 29,98<br>28,5 |

**Stade C :** [Trans (±)] 3,4-dichloro N-[2,3-dihydro 2-(4-méthyl 1-pipérazinyl) 1H-indèn-1-yl] N-méthyl benzène acétamide.

On opère comme au stade C de l'exèmple 23 à partir de 0,720 g de chlorhydrate obtenu au stade B ci-dessus et 0,533 g d'acide 3,4-dichlorophényl acétique. On obtient 1,070 g de produit attendu sous forme de base puis 0,886 g de chlorhydrate. F = 223¤C.

| Analyse : $C_{23}H_{27}Cl_2N_3O$, 2HCl : 505,318 | | | | |
|---|---|---|---|---|
| Calculé :<br>Trouvé : | C% 54,67<br>54,5 | H% 5,78<br>5,9 | N% 8,31<br>8,2 | Cl% 28,06<br>27,5 |

**Exemple 25 : [Trans (±)] 3,4-dichloro N-[2,3-dihydro 2-(diméthylamino) 1H-indèn-1-yl] N-méthyl benzène acétamide et son chlorhydrate.**

**Stade A :** [Trans (±)] 2,3-dihydro 1-(diméthylamino) 1H-indèn-2-ol et son chlorhydrate.

On opère comme au stade A de l'exemple 23 mais après l'extraction à l'éther et l'élimination des solvants sous pression réduite, on reprend le résidu dans l'éther, ajoute 70 cm3 d'une solution éthanolique de chlorure d'hydrogène (1,68 N), amorce la cristallisation, abandonne 2 heures, essore et rince le produit à l'éthanol puis à l'éther. On obtient 15,15 g de produit attendu que l'on recristallise dans l'isopropanol. F = 184¤C.

**Stade B :** [Trans (±)] 2,3-dihydro N-méthyl 2-(diméthylamino)1H-indèn-1-amine et son oxalate.

On opère comme au stade B de l'exemple 23 au départ de 4,27 g du chlorhydrate obtenu au stade A ci-dessus et obtient 3,87 g de produit attendu sous forme de base.

Formation de l'oxalate.

On dissout 3,82 g de base dans 2 cm3 d'éthanol, ajoute à chaud 2,67 g d'acide oxalique, essore, rince à l'éthanol puis à l'éther et recueille 3,22 g d'oxalate attendu. F = 170¤C.

| Analyse : | | | |
|---|---|---|---|
| Calculé :<br>Trouvé : | C% 55,37<br>55,2 | H% 6,50<br>6,5 | N% 8,61<br>8,7 |

**Stade C :** [Trans (±)] 3,4-dichloro N-[2,3-dihydro 2-(diméthylamino) 1H-indèn-1-yl] N-méthyl benzène acétamide et son chlorhydrate.

On opère comme au stade C de l'exemple 23 au départ de 0,650 g de l'oxalate obtenu au stade B ci-dessus et 0,533 g d'acide 3,4-dichlorophényl acétique. On obtient 0,834 g de produit attendu sous forme de base puis 0,683 g de chlorhydrate. F # 233¤C (décomposition).

| Analyse : $C_{20}H_{22}Cl_2N_2O$, HCl : 413,777 | | | | |
|---|---|---|---|---|
| Calculé :<br>Trouvé : | C% 58,06<br>58,2 | H% 5,60<br>5,6 | N% 6,77<br>6,7 | Cl% 25,70<br>25,9 |

**Exemple 26 : [Trans (±)] 3,4-dichloro N-[2,3-dihydro 1,1-diméthyl 2-(1-pyrrolidinyl) 1H-indèn-3-yl] N-méthyl benzène acétamide et son trans butène dioate.**

**Stade A :** Oxyde de diméthyl 1,1-indène.

On refroidit à 7¤C, 5 g de 1,1-diméthyl indène préparé comme dans "Boschond and R.K. Brown, Canadian J. of Chem. 42, 1718 (1964), dans 40 cm3 de chlorure de méthylène, 75 cm3 d'une solution aqueuse à 10% de bicarbonate de soude et 5,45 g de bicarbonate de soude et ajoute 11,57 g d'acide métachloroperbenzoïque en maintenant la température inférieure à 10¤C. On agite ensuite 22 heures à température ambiante, filtre, décante, lave la phase aqueuse au chlorure de méthylène, réunit les phases organiques, les lave avec une solution aqueuse à 10% de thiosulfate de sodium puis avec une solution aqueuse à 10% de bicarbonate de soude puis à l'eau, sèche et élimine les solvants sous pression réduite et obtient 9,2 g de produit attendu que l'on utilise tel quel dans le stade suivant.

**Stade B :** [Trans (±)] 1,1-diméthyl 3-pyrrolidine 2-indanol.

On ajoute le mélange de 7 cm3 de pyrrolidine et 7 cm3 d'eau à 9 g du produit obtenu au stade A ci-dessus, puis chauffe à 65¤C pendant 2 heures le milieu réactionnel. On refroidit, dilue par 10 cm3 d'eau, élimine l'excès de pyrrolidine sous pression réduite, extrait à l'acétate d'éthyle, sèche et concentre à sec sous pression réduite. On obtient 10 g de produit brut que l'on purifie par chromatographie sur silice (éluant : n-hexane-acétace d'éthyle-triéthylamine (27-70-3). F = 86¤C.

**Stade C :** Mésylate de [trans (±)] 1,1-diméthyl 3-pyrrolioine 2-indanol.

On refroidit à -20¤C, 4,8 g de produit préparé au stade ci-dessus, 50 cm3 de tétrahydrofuranne et 3,86 cm3 de triéthylamine et ajoute goutte à goutte 1,8 g de chlorure de méthane sulfonyl en solution dans 5 cm3 de tétrahydrofuranne. On laisse revenir à température ambiante et maintient 64 heures sous agitation. On dilue à l'eau glacée, ajoute 2 cm3 de soude 0,1 N, extrait à l'acétate d'éthyle, lave la phase organique à l'eau salée, sèche et élimine les solvants sous pression réduite. On obtient 6,9 g de produit utilisé tel quel pour le stade suivant.

**Stade D :** [Trans (±)] 2,3-dihydro N-méthyl 2-(1-pyrrolidinylà 3,3-diméthyl 1H-indèn-1-amine.

On chauffe pendant 18 heures à 70¤C (P = 1,5 barr) dans un autoclave, 6,9 g du produit obtenu au stade précédent dans 15 cm3 d'une solution aqueuse de méthylamine à 35-40%. Après refroidissement, on dilue à l'eau salée, extrait à l'acétate d'éthyle, lave la phase organique à l'eau, sèche et concentre sous pression réduite. On obtient 6 g de produit brut que l'on purifie par chromatographie sur silice (éluant : acétate d'éthyle-n-hexane-triéthylamine 60-37-3).

**Stade E :** [Trans (±)] 3,4-dichloro N-[2,3-dihydro 1,1-diméthyl 2-(1-pyrrolidinyl) 1H-indèn-3-yl] N-méthyl benzène acétamide et son trans butène dioate.

On opère comme au stade D de l'exemple 2 au départ de 0,570 g du produit préparé au stade C ci-dessus et 0,668 g d'acide 3,4-dichlorophényl acétique. On obtient 1,232 g de produit attendu sous forme de base. 1,064 g de cette base sont transformés en fumarate. F = 170¤C.

| Analyse : $C_{24}H_{28}Cl_2N_2O$, $C_4H_4O_4$ 547,483 | | | | |
|---|---|---|---|---|
| Calculé :<br>Trouvé : | C% 61,43<br>61,3 | H% 5,89<br>6,0 | N% 5,12<br>5,1 | Cl% 12,95<br>12,9 |

**Exemple 27 : [Trans (±)] N-[2,3-dihydro 1,1-diméthyl 2-(1-pyrrolidinyl) 1H-indèn-3-yl] N-méthyl 4-nitro benzène acétamide et son trans butène dioate.**

On opère comme au stade D de l'exemple 2 au départ de 0,489 g de [trans (±)] 2,3-dihydro N-méthyl 2-(1-pyrrolidinyl) 3,3-diméthyl 1H-indèn-1-amine préparé à l'exemple précédent stade D et 0,471 g d'acide p-nitro phényl acétique. On obtient 0,888 g de produit attendu sous forme de base puis 0,433 g de fumarate. F = 161¤C.

| Analyse : $C_{24}H_{29}N_3O_3$, 1,25 $C_4H_4O_4$ 552,602 | | | |
|---|---|---|---|
| Calculé :<br>Trouvé : | C% 63,03<br>62,8 | H% 6,20<br>6,3 | N% 7,60<br>7,5 |

**Exemple 28 : [Trans (±)] N-[2,3-dihydro 1,1-diméthyl 3-(1-pyrrolidinyl) 1H-indèn-2-yl] N-méthyl 4-nitro benzène acétamide et son chlorhydrate.**

**Stade A :** [Trans (±)] 2,3-dihydro 1-[méthyl (phénylméthyl) amino] 3,3-diméthyl 1H-indèn-2-ol.

On ajoute 10,4 cm3 de N-benzylméthylamine dans 50 cm3 d'eau et 12,4 g d'oxyde de 1,1-diméthyl indène obtenu comme au stade A de l'exemple 26, et chauffe le mélange 3 heures à 90¤/95¤C. On refroidit à +5¤C, élimine par décantation la phase aqueuse, reprend la phase organique à l'acétate d'éthyle et extrait avec une solution aqueuse d'acide chlorhydrique 1 N. On neutralise avec une solution aqueuse saturée en bicarbonate de soude, extrait à l'acétate d'éthyle, sèche et élimine les solvants sous pression réduite. On obtient 10 g de produit brut que l'on purifie par chromatographie sur silice (éluant : n-hexane-acétate d'éthyle 8-2) et recueille 8,86 g de produit attendu.

**Stade B :** Mésylate de [trans (±)] 1,1-diméthyl 3-benzylméthylamino 2-indanol.

On refroidit à -20¤C, 8,7 g du produit obtenu au stade A ci-dessus dans 80 cm3 de chlorure de méthylène et 13 cm3 de triéthylamine et ajoute goutte à goutte 4,85 cm3 de chlorure de méthane sulfonyle dans 24 cm3 de chlorure de méthylène. On agite 30 minutes à -20¤C, laisse remonter la température à +3¤C, ajoute de l'eau froide, sépare la phase organique, la lave à l'eau, la sèche et élimine les solvants sous pression réduite. On obtient 13 g de produit attendu que l'on utilise tel quel dans le stade suivant.

**Stade C :** [Trans (±)] 1,1-diméthyl 2-benzylméthylamino 3-pyrrolidino indane.

On ajoute à température ambiante 30 cm3 de pyrrolidine dans 30 cm3 d'eau distillée à 13 g de produit obtenu au stade B ci-dessus. On chauffe 17 heures à 75¤C, refroidit à température ambiante, évapore l'excès de pyrrolidine sous pression réduite, sature le milieu réactionnel en chlorure de sodium, extrait à l'acétate d'éthyle, sèche et élimine les solvants sous pression réduite. On obtient 13,5 g de produit brut que l'on purifie par chromatographie sur silice (éluant : n-hexane-acétate d'éthyle 8-2). On recueille 8,7 g de produit attendu.

**Stade D :** [Trans (±)] 1,1-diméthyl 2-méthylamino 3-pyrrolidino indane.

On hydrogène pendant 14 heures (p = 185 mbar) 2,5 g de produit obtenu au stade précédent dans 56,5 cm3 de méthanol, 4 cm3 d'acide chlorhydrique à 37% en présence de 1,7 g de charbon actif à 10% de palladium. On évapore le méthanol sous pression réduite, reprend à l'eau, neutralise avec de la triéthylamine, extrait à l'acétate d'éthyle, sèche et élimine les solvants sous pression réduite. On reprend 0,7 g de résidu dans 5 cm3 d'eau, ajoute 6 cm3 de lessive de soude à 32%, extrait à l'acétate d'éthyle, lave avec une solution aqueuse de chlorure de sodium, sèche et concentre à sec sous pression réduite. On obtient 0,64 g de produit attendu que l'on utilise tel quel pour le stade suivant.

**Stade E :** [Trans (±)] N-[2,3-dihydro 1,1-diméthyl 3-(1-pyrrolidinyl) 1H-indèn-2-yl] N-méthyl 4-nitro benzène acétamide et son chlorhydrate.

On opère comme au stade C de l'exemple 1 à partir de 0,386 g de produit obtenu au stade D ci-dessus et 0,371 g d'acide p-nitrophényl acétique. On obtient après chromatographie du produit brut sur silice (éluant : acétate d'éthyle à 1% de triéthylamine) 0,585 g de produit attendu sous forme de base puis 0,483 g de chlorhydrate. F = 209¤C.

| Analyse : $C_{24}H_{29}N_3O_3$, HCl : 443,97 | | | | |
|---|---|---|---|---|
| Calculé :<br>Trouvé : | C% 64,92<br>64,9 | H% 6,81<br>6,9 | N% 9,46<br>9,5 | Cl% 7,98<br>7,8 |

**Exemple 29 : [Trans (±)] 3,4-dichloro N-[2,3-dihydro 1,1-diméthyl 3-(1-pyrrolidinyl) 1H-indèn-2-yl] N-méthyl benzène acétamide et son trans butène dioate.**

On opère comme au stade D de l'exemple 2 à partir de 0,570 g de produit obtenu au stade D de l'exemple 28 et 0,668 g d'acide 3,4-dichloro phényl acétique et obtient 1,232 g de produit attendu sous forme de base puis 0,502 g de fumarate. F = 170¤C.

| Analyse : $C_{24}H_{28}Cl_2N_2O$, $C_4H_4O_4$ : 547,483 | | | | |
|---|---|---|---|---|
| Calculé :<br>Trouvé : | C% 61,43<br>61,3 | H% 5,89<br>6,0 | N% 5,12<br>5,0 | Cl% 12,95<br>12,9 |

En opérant selon un mode opératoire indiqué ci-dessus, on a préparé les produits des exemples 30 et 31 suivants.

**Exemple 30 : [Trans (±)] N-[4-bromo 2,3-dihydro 2-(1-pyrrolidinyl) 1H-indèn-1-yl] 3,4-dichloro N-méthyl benzène acétamide et son chlorhydrate.**

**Stade A :** 3-bromo 1aH-indeno [1,2-b] oxizène.

On opère comme au stade A de l'exemple 26 au départ de 13,2 g de 7-bromo 1H-indène et obtient 17,32 g de l'époxyde que l'on utilise tel quel pour le stade suivant.

**Stade B :** [Trans (±)] 4-bromo 2,3-dihydro 1-(1-pyrrolidinyl) 1H-inden-2-ol et son chlorhydrate.

En opérant comme au stade B de l'exemple 26 à partir du produit obtenu ci-dessus, on obtient 7,88 g de produit brut que l'on transforme en chlorhydrate à l'aide de 16 cm3 d'une solution éthanolique de chlorure d'hydrogènr (1,68 N). F = 229¤C (décomposition).

**Stade C :** [Trans (±)] 4-bromo 2,3-dihydro N-méthyl 2-(1-pyrrolidinyl) 1H-indèn-1-amine et son chlorhydrate.

En opérant comme aux stades C et D de l'exemple 26 à partir du produit obtenu ci-dessus, on obtient 4,12 g de produit brut sous forme de base que l'on transforme en chlorhydrate à l'aide de 4 cm3 d'une

solution éthanolique de chlorure d'hydrogène (≃ 6,6 N). Onobtient 3,82 g de chlorhydrate. F # 240¤C.

**Stade D :** [Trans (±)] N-[4-bromo 2,3-dihydro 2-(1-pyrrolidinyl) 1H-indèn-1-yl] 3,4-dichloro N-méthyl benzène acétamide et son chlorhydrate.

On opère comme au stade C de l'exemple 1 à partir de 0,533 g d'acide 3,4-dichlorophényl acétique, 0,421 g de carbonyldiimidazole et 0,736 g de chlorhydrate obtenu au stade C précédent. On obtient 0,876 g de produit sous forme de base puis 0,587 g de chlorhydrate. F = 208¤C.

| Analyse : $C_{22}H_{23}BrCl_2N_2O$, HCl : 518,716 | | | | | |
|---|---|---|---|---|---|
| Calculé :<br>Trouvé : | C% 50,94<br>51,1 | H% 4,66<br>4,6 | N% 5,40<br>5,3 | Cl% 20,50<br>20,3 | Br% 15,40<br>15,4 |

**Exemple 31: [Trans (±)] N-[5-chloro 2,3-dihydro 2-(1-pyrrolidinyl) 1-indèn-1-yl] 3,4-dichloro N-méthyl benzène acétamide et son chlorhydrate.**

**Stade A :** [Trans (±)] 4-chloro 1aH-indeno [1,2-b] oxirène.

On opère comme au stade A de l'exemple 26 à partir de 10,05 g de 6-chloro 1H-indène et obtient 14,2 g de produit attendu que l'on utilise tel quel pour le stade suivant.

**Stade B :** [Trans (±)] 5-chloro 2,3-dihydro 1-(1-pyrrolidinyl) 1H-indèn-2-ol et son chlorhydrate.

On opère comme au stade B de l'exemple 26 à partir du produit obtenu ci-dessus et obtient 4,48 g de produit attendu sous forme de base que l'on transforme en chlorhydrate à l'aide de 11 cm3 d'une solution éthanolique de chlorure d'hydrogène (0,68 N) et obtient 3,52 g de chlorhydrate attendu. F = 159¤C.

**Stade C :** [Trans (±)] 5-chloro 2,3-dihydro N-méthyl 2-(1-pyrrolidinyl) 1H-indèn-1-amine et son oxalate.

En opérant comme aux stades C et D de l'exemple 26 à partir du produit obtenu précédemment. On obtient 3,83 g de produit attendu sous forme de base puis 4,38 g d'oxalate par addition de 2,3 g d'acide oxalique dihydraté. F # 165¤C.

**Stade D :** [Trans (± ] N-[5-chloro 2,3-dihydro 2-(1-pyrrolidinyl) 1H-indèn-1-yl] 3,4-dichloro N-méthyl benzène acétamide et son chlorhydrate.

On opère comme au stade C de l'exemple 1, à partir de 0,533 g d'acide 3,4-dichlorophényl acétique, 0,421 g de carbonyldiimidazole et 0,772 g de l'oxalate obtenu au stade C précédent. On obtient 0,940 g de produit attendu sous forme de base puis 0,760 g de chlorhydrate. F = 228¤C.

| Analyse : $C_{22}H_{23}Cl_3N_2O$, HCl : 474,26 | | | | |
|---|---|---|---|---|
| Calculé :<br>Trouvé : | C% 55,72<br>56,0 | H% 5,10<br>5,2 | N% 5,91<br>5,9 | Cl% 22,90<br>29,6 |

**Exemples de composition pharmaceutique.**

**Exemple 32 :**

On a préparé des comprimés répondant à la formule suivante :

| | |
|---|---|
| - produit de l'exemple 1<br>- excipient q.s.p. | 200 mg<br>800 mg. |
| (détail de l'excipient : lactose, talc, amidon, stéarate de magnésium). | |

**Exemple 33 :**

On a préparé un soluté injectable (voie intra-musculaire) répondant à la formule suivante :

| | |
|---|---|
| - produit de l'exemple 25<br>- solvant stérile q.s.p. | 50 mg<br>5 cm3. |

**ETUDE PHARMACOLOGIOUE DU PRODUIT**

1) Liaison au récepteur opiacé K in vitro.

On utilise des culots membranaires conservés à -30¤C (éventuellement pendant environ 30 jours) et préparés à partir de cervelets de cobayes.

Ces culots sont remis en suspension dans le tampon Tris pH 7,7. On répartit les fractions de 2 cm3 dans des tubes à hémolyse et ajoute de la $9^3$H éthylkétocyclazocine 1nM et le produit à étudier. (Le produit est d'abord testé à $5\times10^{-6}$M (en triple). Lorsque le produit testé déplace de plus de 50% la radioactivité liée spécifiquement au récepteur, il est testé à nouveau selon une gamme de 7 doses afin de déterminer la dose qui inhibe de 50% la radioactivité liée spécifiquement au récepteur. On détermine ainsi la concentration inhibitrice 50%).

La liaison non spécifique est déterminée par addition de produit connu sous le nom U-50488 H (Lahti et al. 1982, Life Sci. 31, 2257) à $10^{-5}$M (en triple). On incube à 25¤C pendant 40 minutes, remet au bain-marie à 0¤C, 5 minutes, filtre sous vide, rince au tampon Tris pH 7,7 et compte la radioactivité en présence de scintillant Trition.

Le résultat est exprimé directement en concentration inhibitrice 50% ($CI_{50}$), (c'est-à-dire en concentration de produit étudié, exprimée en nM, nécessaire pour déplacer 50% de la radioactivité spécifique fixée sur le récepteur étudié.

Résultats :

| Produit de l'exemple | $CI_{50}$ en nM |
|---|---|
| 1 | 1,8 |
| 5 | 3 |
| 6 | 7,5 |
| 8 | 7,1 |
| 9 | 2,7 |
| 14 | 3,8 |
| 16 | 5 |
| 22 | 7,1 |
| 25 | 2,2 |
| 29 | 5,6 |

2) Activité analgésique.

- Test de la plaque chaude.

Des souris femelles pesant 22 à 24 g sont placées une par une sur une plaque de cuivre maintenue à 56¤C : la réaction à la douleur se manifeste par le léchage des pattes avant de l'animal ; le temps de cette réaction est noté et l'on ne retient que les souris réagissant en moins de 8 secondes.

Les animaux sont répartis par groupes homogènes et traités par le produit à étudier administré par voie buccale, un groupe ne recevant que le véhicule. Le temps de réaction à la douleur est de nouveau mesuré 30 à 60 minutes après le traitement. La dose active ou DA100 est la dose qui augmente le temps de réaction de 100%, 30 minutes après le traitement compte tenu des variations du temps de réaction des animaux témoins.

Pour le produit de l'exemple 1, la $DA_{100}$ est de 50 mg/kg.

3) Etude de l'activité hypotensive sur le rat normotendu anesthésié.

Des rats mâles Sprague Dawley (CR) sont anesthésiés par voie intrapéritonéale au pentobarbital sodique (60 mg/kg).

Une veine jugulaire est cathétérisée pour l'injection du produit, et une artère carotide est cathétérisée pour l'enregistrement de la pression artérielle.

Le produit à tester est mis en solution dans 10% d'éthanol puis injecté sous un volume de 1 cm3/kg.

La pression est notée aux temps 5 minutes et 30 minutes après l'injection du produit.

Le tableau ci-après indique les variations exprimées en pourcentage de la pression artérielle après administration du produit testé par rapport à la pression artérielle témoin initiale.

Résultats :

| Produit de l'exemple | dose mg/kg | 5 mn après administration | 30 mn après administration |
|---|---|---|---|
| 1 | 10 | - 13 | - 19 |
| | 1 | - 19 | - 23 |
| 4 | 10 | - 26 | - 14 |
| 5 | 10 | - 25 | - 10 |
| | 1 | - 20 | - 20 |
| 8 | 1 | - 25 | - 27 |
| 14 | 1 | - 22 | - 26 |
| 9 | 10 | - 32 | - 31 |

4) Mesure de l'activité diurétique.

Des rats mâles, de souche Sprague Dawley et de poids 180-200 g, sont mis à jeun 17 heures avant l'essai, tout en recevant de l'eau ad libitum.

Des lots de 8 animaux sont constitués par dose testée. Les rats reçoivent le produit à tester ou son véhicule par voie orale.

Le volume urinaire est mesuré toutes les heures pendant les 5 heures qui suivent l'administration du produit. A la fin de cette période, les urines sont recueillies et l'activité du produit est exprimée en pourcentage de variation calculé sur le volume urinaire correspondant à la période $t_{1h-5h}$.

Les résultats obtenus sont les suivants :

| Produit de l'exemple | dose mg/kg | pourcentage de variation du volume urinaire |
|---|---|---|
| 1 | 5 | + 188 |
| 5 | 5 | + 190 |
| 8 | 5 | + 130 |
| | 2,5 | + 124 |
| 14 | 5 | + 68 |
| 9 | 10 | + 74 |

5) Action antiarythmique chez le rat.

On trachéotomise des rats mâles pesant 300-350 g anesthésiés par voie intrapéritonéale à l'aide de 1,20g/kg d'uréthane et les soumet à une respiration artificielle (40-50 insufflations de 3 ml/minute).

On implante des aiguilles en sous cutané de manière à enregistrer l'électrocardiogramme des rats sur le signal en dérivation DII.

On administre le produit à tester par voie intraveineuse.

Cinq minutes après l'administration du produit, on perfuse la veine jugulaire des rats avec 10 µg/nm sous 0,2 ml d'une solution d'aconitine et on note le temps d'apparition des troubles du rythme cardiaque.

Les résultats sont exprimés en pourcentage d'allongement du temps d'apparition des troubles du rythme cardiaque par rapport aux témoins et en fonction de la dose du produit testé.

Les résultats figurant sur le tableau ci-après montrent que certains des produits de la présente demande sont doués de bonnes propriétés antiarythmiques.

| Produit de l'exemple | Dose mg/kg | Pourcentage d'allongement du temps |
|---|---|---|
| 1 | 10 | + 31,4 |
| | 5 | + 20,6 |
| 5 | 10 | + 15,6 |
| 8 | 5 | + 23 |
| 9 | 10 | + 34,6 |
| | 5 | + 32 |

## Revendications

1. Composés de formule (I) :

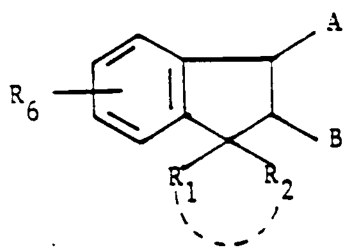

(I)

dans laquelle $R_6$ représente un atome d'hydrogène, un atome d'halogène, un radical alkyle ou alcoxy renfermant de 1 à 5 atomes de carbone et dans laquelle $R_1$ et $R_2$, identiques ou différents, représentent un atome d'hydrogène ou un radical alkyle renfermant de 1 à 5 atomes de carbone ou $R_1$ et $R_2$ forment ensemble avec l'atome de carbone auquel ils sont liés un cycle cycloalkyle renfermant entre 3 et 6 atomes de carbone et pouvant contenir éventuellement un hétéroatome tel et qu'un atome d'oxygène ou de soufre ou d'azote et dans laquelle A et B sont tels que l'un des substituants A ou B est choisi dans

- le groupe de formule

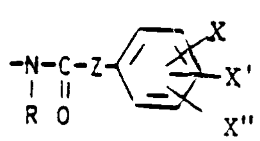

R étant un atome d'hydrogène ou un radial alkyle renfermant de 1 à 5 atomes de carbone, Z une chaîne alkylène linéaire $-(CH_2)_n-$, n étant un nombre entier pouvant varier entre 0 et 5 ou une

chaîne alkylène ramifiée renfermant de 2 à 8 atomes de carbone ou un groupement $-CH_2-O-$, X, X' X", identiques ou différents, représentent un atome d'hydrogène, un radical alkyle ou alkoxy renfermant de 1 à 4 atomes de carbone, un atome d'halogène, un radical hydroxyle, trifluorométhyle, nitro, amino, monoalkyl ou dialkyl-amino ou sulfoamino et l'autre des substituants A ou B est choisi dans

- le groupe de formule :

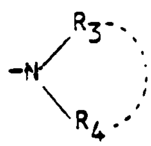

dans lequel $R_3$ et $R_4$, identiques ou différents, représentent un atome d'hydrogène ou un radial alkyle renfermant de 1 à 5 atomes de carbone ou $R_3$ et $R_4$ forment ensemble avec l'atome d'azote auquel ils sont liés un hétérocycle à 5 ou 6 chaînons comportant éventuellement un autre hétéroatome tel que l'oxygène, l'azote ou le soufre, l'atome d'azote étant éventuellement substitué par un radical alkyle renfermant de 1 à 4 atomes de carbone étant entendu que $R_6$ ne représente pas un radical méthoxy quand $R_1$ et $R_2$ représentent un atome d'hydrogène, B représente un radical pyrrolidinyle et A représente le radical 2,3-dichloro N-méthyl benzèneacétamide, lesdits composés de formule (I) pouvant être dans toutes les formes enantiomères et diastéréisomères possibles et sous forme de sels d'addition avec les acides.

**2.** Composés de formule (I), tels que définis à la revendication 1, caractérisés en ce que les groupements A et B sont en configuration trans ainsi que leurs sels d'addition avec les acides.

**3.** Composés de formule (I), tels que définis aux revendications 1 et 2, caractérisés en ce que dans le groupe

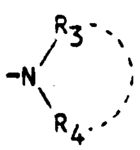
,

$R_3$ et $R_4$ représentent tous deux un radical méthyle ou forment avec l'atome d'azote auquel ils sont liés l'hétérocycle pyrrolidine, pipéridine, ou pipérazine éventuellement substitué par un radical alcoyle renfermant de 1 à 3 atomes de carbone, ainsi que leurs sels d'addition avec les acides.

**4.** Composés de formule (I), tels que définis aux revendications 1 à 3, caractérisés en ce que dans le groupe

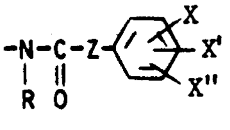
,

R représente un atome d'hydrogène, un radical méthyle ou éthyle, Z un groupe $-(CH_2)_n-$ dans lequel n est 0 ou 1,

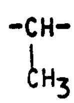

ou $-CH_2-O-$, ainsi que leurs sels d'addition avec les acides.

**5.** Composés de formule (I), tels que définis aux revendications 1 à 4, caractérisés en ce que $R_1$ et $R_2$ représentent tous deux un atome d'hydrogène, un radical méthyle ou forment ensemble avec l'atome de carbone auquel ils sont liés le cycle tétrahydropyranne ainsi que leurs sels d'addition avec les acides.

**6.** Composés de formule (I), tels que définis aux revendications 1 à 5, caractérisés en ce que X, X′ et X″, identiques ou différents, représentent un atome d'hydrogène, un radical méthyle ou éthyle, méthoxy ou éthoxy, nitro, sulfoamino, trifluorométhyle, un atome de chlore ainsi que leurs sels d'addition avec les acides.

**7.**

- le [trans (±)] 3,4-dichloro N-[2,3-dihydro 2-(1-pyrrolidinyl) 1H-indèn-1-yl] N-méthyl benzène acétamide,
- le [trans (±)] N-[2,3-dihydro 2-(1-pyrrolidinyl) 1H-indèn-1-yl] N-méthyl 3-nitro benzène acétamide,
- le [trans (±)] N-[2,3-dihydro 2-(1-pyrrolidinyl) 1H-indèn-1-yl] N-méthyl 4-nitro benzène acétamide,
- le [trans (±)] N-[2,3-dihydro 2-(1-pyrrolidinyl) 1H-indèn-1-yl] N-méthyl (4-trifluorométhyl) benzène acétamide,
- le [trans (±)] 2-(3,4-dichlorophénoxy) N-[2,3-dihydro 2-(1-pyrrolidinyl) 1H-indèn-1-yl] N-méthyl acétamide,
- le [trans (±)] 3,4-dichloro N-[2,3-dihydro 2-(diméthylamino) 1H-indèn-1-yl] N-méthyl acétamide,
- le [trans (±)] N-[2,3-dihydro 2-(1-pyrrolidinyl) 1H-inden-1-yl] N-méthyl 4-nitro benzène acétamide (isomère A) ainsi que leurs sels d'addition avec les acides.

**8.** Procédé de préparation des produits de formule (I) dont les groupements A et B sont en configuration trans, caractérisé en ce que l'on condense le produit de formule (II) :

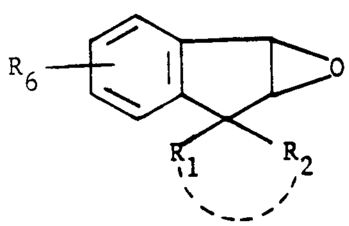

(II)

$R_6$, $R_1$ et $R_2$ ayant toutes les significations données précédemment,

- soit avec une amine de formule (III) :

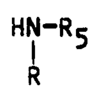

(III)

R ayant toutes les significations données précédemment et $R_5$ étant un groupement protecteur de la fonction amine et notamment un groupement benzyle, pour obtenir un produit de formule (IV) :

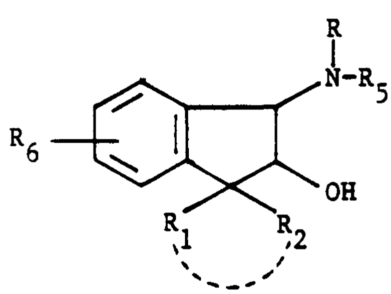

(IV)

dont on active la fonction hydroxyle et que l'on condense avec une amine de formule (V) :

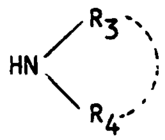

(V)

dans laquelle $R_3$ et $R_4$ ont les significations données précédemment pour obtenir un produit de formule (VI) :

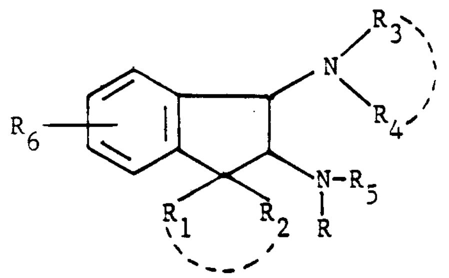

(VI)

dont on élimine le groupement protecteur $R_5$ de la fonction amine pour obtenir un produit de formule (VII) :

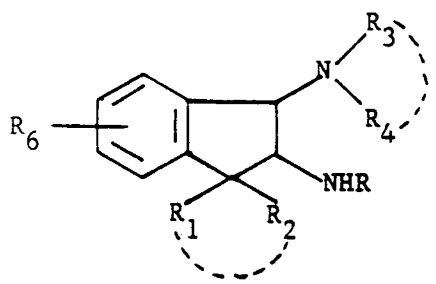

(VII)

que l'on traite avec un acide de formule (VIII) ou un dérivé fonctionnel de cet acide :

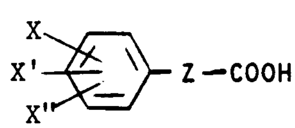

(VIII)

dans laquelle Z, X, X′ et X″ ont toutes les significations précédentes pour obtenir un produit de formule (I) dans laquelle A représente le groupe

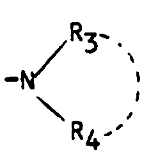 et B le groupe 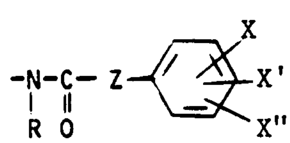 ,

- soit avec une amine de formule (V) :

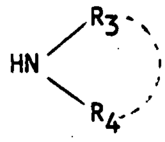

(V)

pour obtenir un produit de formule (IX) :

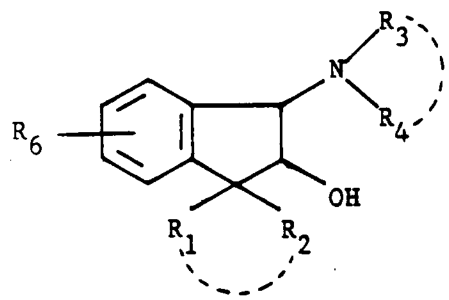

(IX)

dont on active la fonction hydroxyle et que l'on traite avec une amine de formule (X) :

$NH_2R$ (XI)

dans laquelle R a la signification précédente pour obtenir un produit de formule (XI) :

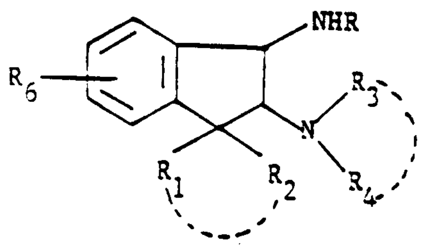

(XI)

que l'on condense avec un acide de formule (VIII) ou un dérivé fonctionnel de cet acide :

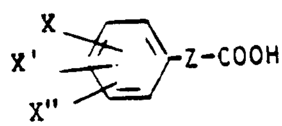

dans laquelle Z, X, X' et X" ont les significations précédentes pour obtenir un produit de formule (I) dans laquelle A représente le groupe

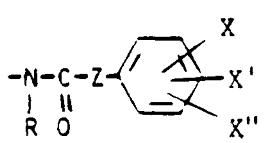 et B le groupe 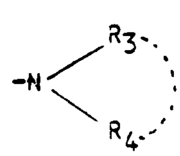 ,

lesdits composés de formule (I) pouvant être dédoublés pour obtenir les formes optiquement actives et que l'on traite, si désiré, avec un acide minéral ou organique, pour obtenir un sel.

**9.** A titre de médicaments, les produits de formule (I), tels que définis à l'une quelconque des

revendications 1 à 6, ainsi que leurs sels d'addition avec les acides pharmaceutiquement acceptables.

**10.** A titre de médicaments, les produits de la revendication 7, ainsi que leurs sels d'addition avec les acides pharmaceutiquement acceptables.

**11.** Les compositions pharmaceutiques contenant à titre de principe actif, l'un au moins des médicaments tels que définis par la revendication 9 ou 10.

**12.** A titre de produits intermédiaires, les produits de formule (IV), (VI), (VII), (IX), (XI) dans laquelle $R_6$ ne représente pas un atome d'hydrogène et R1 et R2 ne représentent pas un atome d'hydrogène ou un radical alkyle renfermant de 1 à 5 atomes de carbone,

**Claims**

**1.** Compounds of formula (I):

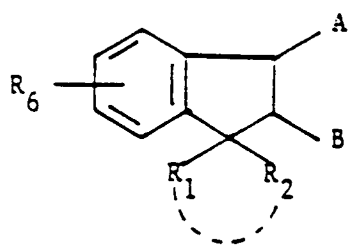

(I)

in which $R_6$ represents a hydrogen atom, a halogen atom, an alkyl or alkoxy radical containing 1 to 5 carbon atoms and in which $R_1$ and $R_2$, identical or different, represent a hydrogen atom or an alkyl radical containing 1 to 5 carbon atoms or $R_1$ and $R_2$ form together with the carbon atom to which they are linked a cycloalkyl ring containing between 3 and 6 carbon atoms and optionally able to contain a heteroatom such as an oxygen or sulphur or nitrogen atom and in which A and B are such that one of the substituents A or B is chosen from

- the group of formula

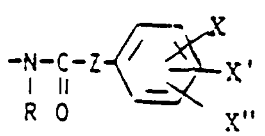

R being a hydrogen atom or an alkyl radical containing 1 to 5 carbon atoms, Z being a $-(CH_2)_n-$ linear alkylene chain, n being an integer which can vary between 0 and 5 or a branched alkylene chain containing 2 to 8 carbon atoms or a $-CH_2-O-$ group, X, X', X", identical or different, represent a hydrogen atom, an alkyl or alkoxy radical containing 1 to 4 carbon atoms, a halogen atom, a hydroxyl, trifluoromethyl, nitro, amino, monoalkyl- or dialkylamino or sulphoamino radical and the other substituent A or B is chosen from

- the group of formula:

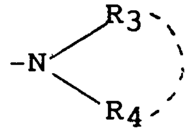

in which $R_3$ and $R_4$, identical or different, represent a hydrogen atom or an alkyl radical containing 1 to 5 carbon atoms or $R_3$ and $R_4$ form together with the nitrogen atom to which they are linked a heterocycle with 5 or 6 links optionally containing another heteroatom such as oxygen, nitrogen or sulphur, the nitrogen atom being optionally substituted by an alkyl radical

containing 1 to 4 carbon atoms, it being understood that $R_6$ does not represent a methoxy radical when $R_1$ and $R_2$ represent a hydrogen atom, B represents a pyrrolidinyl radical and A represents the 2,3-dichloro-N-methyl benzeneacetamide radical, the said compounds of formula (I) being able to be in all the possible enantiomeric and diastereoisomeric forms and in the form of addition salts with acids.

**2.** Compounds of formula (I), as defined in claim 1, characterized in that the groups A and B are in trans configuration, as well as their addition salts with acids.

**3.** Compounds of formula (I), as defined in claims 1 and 2, characterized in that in the group

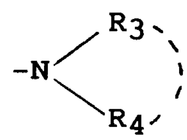 ,

$R_3$ and $R_4$ both represent a methyl radical or form with the nitrogen atom to which they are linked the pyrrolidine, piperidine, or piperazine heterocycle optionally substituted by an alkyl radical containing 1 to 3 carbon atoms, as well as their addition salts with acids.

**4.** Compounds of formula (I), as defined in claims 1 to 3, characterized in that in the group

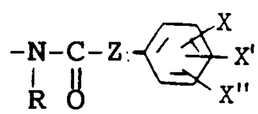 ,

R represents a hydrogen atom, a methyl or ethyl radical, Z a $-(CH_2)_n-$ group in which n is 0 or 1, a

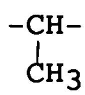

or $-CH_2-O-$ group, as well as their addition salts with acids.

**5.** Compounds of formula (I), as defined in claims 1 to 4, characterized in that $R_1$ and $R_2$ both represent a hydrogen atom, a methyl radical or form with the carbon atom to which they are linked the tetrahydropyran ring, as well as their addition salts with acids.

**6.** Compounds of formula (I), as defined in claims 1 to 5, characterized in that X, X' and X", identical or different, represent a hydrogen atom, a methyl or ethyl, methoxy or ethoxy radical, a nitro, sulphoamino, trifluoromethyl radical, a chlorine atom, as well as their addition salts with acids.

**7.**

- [trans (±)]-3,4-dichloro-N-[2,3-dihydro-2-(1-pyrrolidinyl)-1H-inden-1-yl]-N-methyl benzene acetamide,
- [trans (±)]-N-[2,3-dihydro-2-(1-pyrrolidinyl)-1H-inden-1-yl]-N-methyl-3-nitro benzene acetamide,
- [trans (±)]-N-[2,3-dihydro-2-(1-pyrrolidinyl)-1H-inden-1-yl]-N-methyl-4-nitro benzene acetamide,
- [trans (±)]-N-[2,3-dihydro-2-(1-pyrrolidinyl)-1H-inden-1-yl]-N-methyl-(4-trifluoromethyl)-benzene acetamide,
- [trans (±)]-2-(3,4-dichlorophenoxy)-N-[2,3-dihydro-2-(1-pyrrolidinyl)-1H-inden-1-yl]-N-methyl acetamide,
- [trans (±)]-3,4-dichloro-N-[2,3-dihydro-2-(dimethylamino)-1H-inden-1-yl]-N-methyl acetamide,
- [trans (±)]-N-[2,3-dihydro-2-(1-pyrrolidinyl)-1H-inden-1-yl]-N-methyl-4-nitro benzene acetamide (isomer A), as well as their addition salts with acids.

**8.** Preparation process for products of formula (I) the A and B groups of which are in <u>trans</u> configuration, characterized in that the product of formula (II):

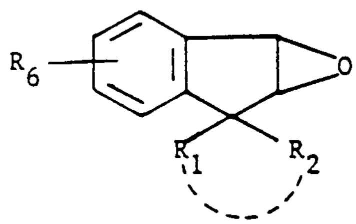

(II)

$R_6$, $R_1$ and $R_2$ having all the meanings given previously, is condensed

- either with an amine of formula (III):

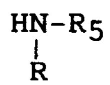

(III)

R having all the meanings given previously and $R_5$ being a protective group of the amine function and in particular a benzyl group, in order to obtain a product of formula (IV):

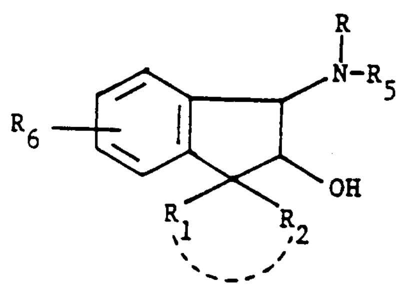

(IV)

the hydroxyl function of which is activated, and which is condensed with an amine of formula (V):

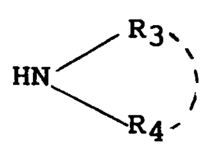

(V)

in which $R_3$ and $R_4$ have the meanings given previously, in order to obtain a product of formula (VI):

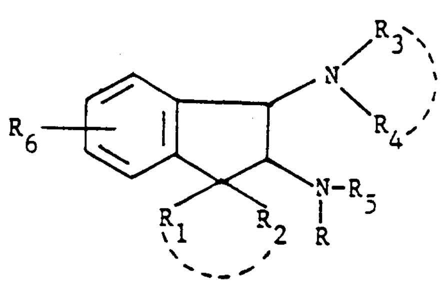

(VI)

the protective group $R_5$ of the amine function of which is eliminated in order to obtain a product of formula (VII):

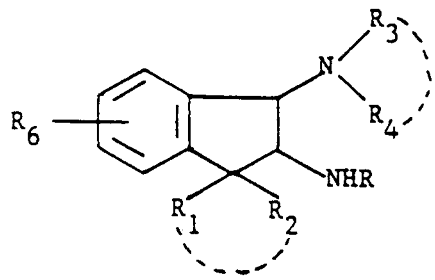

(VII)

which is treated with an acid of formula (VIII) or a functional derivative of this acid:

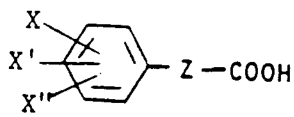

(VIII)

in which Z, X, X' and X" have all the previous meanings, in order to obtain a product of formula (I) in which A represents the group

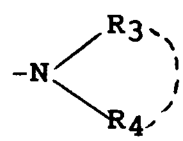

and B the group

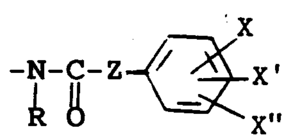

- or with an amine of formula (V):

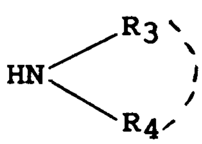

(V)

in order to obtain a product of formula (IX):

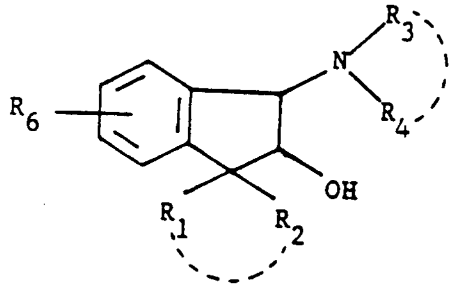

(IX)

the hydroxyl function of which is activated and which is treated with an amine of formula (X):

$NH_2R$ (XI)

in which R has the previous meaning, in order to obtain a product of formula (XI):

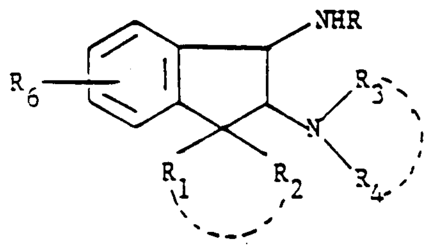

(XI)

which is condensed with an acid of formula (VIII) or a functional derivative of this acid:

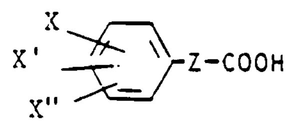

in which Z, X, X' and X" have the previous meanings, in order to obtain a product of formula (I) in which A represents the group

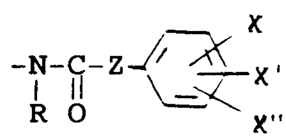

and B the group

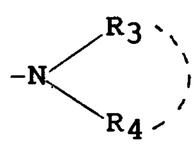

the said compounds of formula (I) being able to be resolved in order to obtain the optically active forms, and which are treated, if desired, with a mineral or organic acid, in order to obtain a salt.

**9.** As medicaments, the products of formula (I), as defined in any one of claims 1 to 6, as well as their addition salts with pharmaceutically acceptable acids.

**10.** As medicaments, the products of claim 7, as well as their addition salts with pharmaceutically acceptable acids.

**11.** The pharmaceutical compositions containing as active ingredient, at least one of the medicaments as defined in claim 9 or 10.

**12.** As intermediate products, the products of formula (IV), (VI), (VII), (IX), (XI) in which $R_6$ does not represent a hydrogen atom and $R_1$ and $R_2$ do not represent a hydrogen atom or an alkyl radical containing 1 to 5 carbon atoms.

**Patentansprüche**

1. Verbindungen der Formel (I)

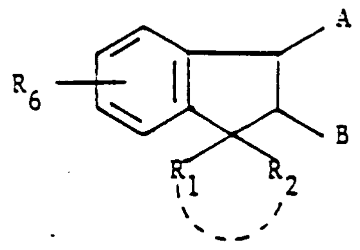

(I)

worin $R_6$ ein Wasserstoffatom, ein Halogenatom, einen Alkyl- oder Alkoxyrest mit 1 bis 5 Kohlenstoffatomen wiedergibt und worin $R_1$ und $R_2$, die gleich oder voneinander verschieden sind, ein Wasserstoffatom oder einen Alkylrest mit 1 bis 5 Kohlenstoffatomen bedeuten oder $R_1$ und $R_2$ gemeinsam mit dem Kohlenstoffatom, an das sie gebunden sind, einen Cycloalkylring mit zwischen 3 und 6 Kohlenstoffatomen bilden, wobei sie gegebenenfalls ein Heteroatom wie ein Sauerstoff- oder Schwefel- oder Stickstoffatom, enthalten können, und worin A und B derart sind, daß einer der Substituenten A oder B ausgewählt ist unter

- der Gruppe der Formel

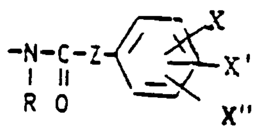

wobei R ein Wasserstoffatom oder einen Alkylrest mit 1 bis 5 Kohlenstoffatomen bedeutet, Z eine lineare Alkylenkette $-(CH_2)_n-$, worin n eine ganze Zahl, die zwischen 0 und 5 variieren kann, bedeutet, oder eine verzweigte Alkylenkette mit 2 bis 8 Kohlenstoffatomen oder eine Gruppe $-CH_2-O-$ wiedergibt, X, X', X", die gleich oder voneinander verschieden sind, ein Wasserstoffatom, einen Alkyl- oder Alkoxyrest mit 1 bis 4 Kohlenstoffatomen, ein Halogenatom, eine Hydroxyl-, Trifluormethyl-, Nitro-, Amino-, Monoalkyl- oder Dialkylamino- oder Sulfoaminogruppe darstellen, und der andere der Substituenten A oder B ausgewählt ist unter

- der Gruppe der Formel

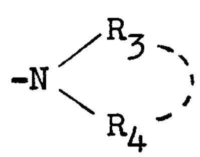

worin $R_3$ und $R_4$, die gleich oder voneinander verschieden sind, für ein Wasserstoffatom oder eine Alkylgruppe mit 1 bis 5 Kohlenstoffatomen stehen oder $R_3$ und $R_4$ gemeinsam mit dem Stickstoffatom, an das sie gebunden sind, einen Heterocyclus mit 5 oder 6 Kettengliedern bilden, der gegebenenfalls ein weiteres Heteroatom, wie Sauerstoff, Stickstoff oder Schwefel, enthält, wobei das Stickstoffatom gegebenenfalls durch eine Alkylgruppe mit 1 bis 4 Kohlenstoffatomen substituiert ist, mit der Maßgabe, daß $R_6$ keine Methoxygruppe bedeutet, wenn $R_1$ und $R_2$ ein Wasserstoffatom wiedergeben, B eine Pyrrolidinylgruppe bedeutet und A den 2,3-Dichlor-N-methylbenzolacetamidrest wiedergibt, wobei die Verbindungen der Formel (I) in sämtlichen möglichen enantiomeren und diastereomeren Formen und in Form der Additionssalze mit Säuren vorliegen können.

2. Verbindungen der Formel (I) gemäß Anspruch 1, dadurch gekennzeichnet, daß die Gruppen A und B sich in trans-Konfiguration befinden, sowie deren Additionssalze mit Säuren.

**3.** Verbindungen der Formel (I) gemäß Anspruch 1 und 2, dadurch gekennzeichnet, daß in der Gruppe

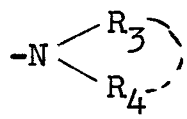

$R_3$ und $R_4$ alle beide eine Methylgruppe wiedergeben oder mit dem Stickstoffatom, an das sie gebunden sind, einen Pyrrolidin-, Piperidin- oder Piperazinheterocyclus bilden, der gegebenenfalls durch einen Alkylrest mit 1 bis 3 Kohlenstoffatomen substituiert ist, sowie deren Additionssalze mit Säuren.

**4.** Verbindungen der Formel (I) gemäß den Ansprüchen 1 bis 3, dadurch gekennzeichnet, daß in der Gruppe

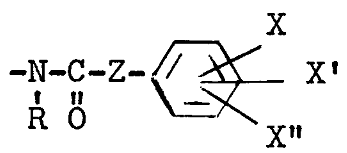

R für ein Wasserstoffatom, eine Methyl- oder Ethylgruppe steht, Z eine Gruppe $-(CH_2)_n-$, worin n für 0 oder 1 steht,

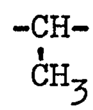

oder $-CH_2-O-$ bedeutet, sowie deren Additionssalze mit Säuren.

**5.** Verbindungen der Formel (I) gemäß den Ansprüchen 1 bis 4, dadurch gekennzeichnet, daß $R_1$ und $R_2$ alle beide ein Wasserstoffatom oder eine Methylgruppe bedeuten oder gemeinsam mit dem Kohlenstoffatom, an das sie gebunden sind, den Tetrahydropyranring bilden, sowie deren Additionssalze mit Säuren.

**6.** Verbindungen der Formel (I) gemäß den Ansprüchen 1 bis 5, dadurch gekennzeichnet, daß X, X' und X", die gleich oder voneinander verschieden sind, für ein Wasserstoffatom, eine Methyl- oder Ethyl-, Methoxy- oder Ethoxy-, Nitro-, Sulfoamino-, Trifluormethylgruppe oder ein Chloratom stehen, sowie deren Addisionssalze mit Säuren.

**7.** Das [trans-(±)]-3,4-Dichlor-N-[2,3-dihydro-2-(1-pyrrolidinyl)-1H-inden-1-yl]-N-methylbenzolacetamid,
das [trans-(±)]-N-[2,3-Dihydro-2-(1-pyrrolidinyl)-1H-inden-1-yl]-N-methyl-3-nitrobenzolacetamid,
das [trans-(±)]-N-[2,3-Dihydro-2-(1-pyrrolidinyl)-1H-inden-1-yl]-N-methyl-4-nitrobenzolacetamid,
das [trans-(±)]-N-[2,3-Dihydro-2-(1-pyrrolidinyl)-1H-inden-1-yl]-N-methyl-(4-trifluormethyl)-benzolacetamid,
das [trans-(±)]-2-(3,4-Dichlorphenoxy)-N-[2,3-dihydro-2-(1-pyrrolidinyl)-1H-inden-1-yl]-N-methylacetamid,
das [trans-(±)]-3,4-Dichlor-N-[2,3-dihydro-2-(dimethylamino)-1H-inden-1-yl]-N-methylacetamid,
das [trans-(±)]-N-[2,3-Dihydro-2-(1-pyrrolidinyl)-1H-inden-1-yl]-N-methyl-4-nitrobenzolacetamid (Isomeres A) sowie deren Additionssalze mit Säuren.

**8.** Verfahren zur Herstellung der Produkte der Formel (I), deren Gruppen A und B sich in der trans-Konfiguration befinden, dadurch gekennzeichnet, daß man das Produkt der Formel (II)

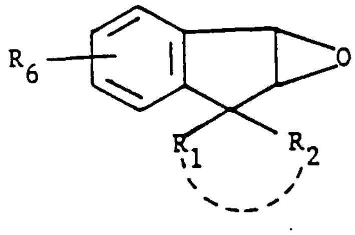

(II)

worin $R_6$, $R_1$ und $R_2$ sämtliche vorstehend angegebenen Bedeutungen besitzen,

- entweder mit einem Amin der Formel (III)

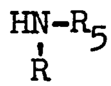

(III)

worin R sämtliche vorstehend angegebenen Bedeutungen besitzt und $R_5$ eine Schutzgruppe für die Aminfunktion und insbesondere eine Benzylgruppe bedeutet, kondensiert, um zu einem Produkt der Formel (IV)

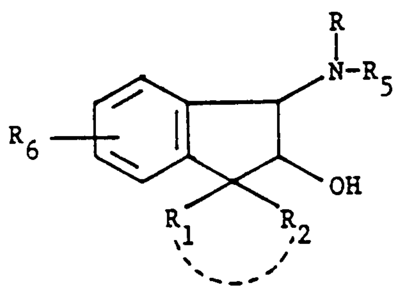

(IV)

zu gelangen, dessen Hydroxylfunktion man aktiviert und welches man mit einem Amin der Formel (V)

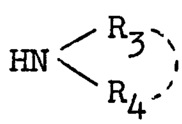

(V)

worin $R_3$ und $R_4$ die vorstehend angegebenen Bedeutungen besitzen, kondensiert, um zu einem Produkt der Formel (VI)

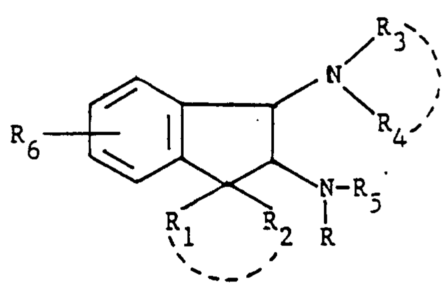

(VI)

zu gelangen, dessen Schutzgruppe $R_5$ der Aminfunktion man entfernt, um zu einem Produkt der Formel (VII)

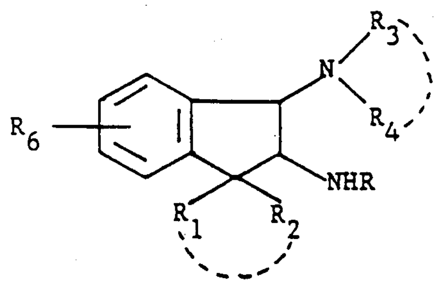

(VII)

zu gelangen, das man mit einer Säure der Formel (VIII) oder einem funktionellen Derivat dieser Säure

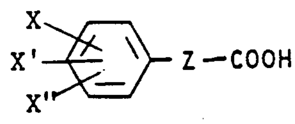

(VIII)

worin Z, X, X' und X" sämtliche vorstehenden Bedeutungen besitzen, behandelt, um zu einem Produkt der Formel (I) zu gelangen, worin A die Gruppe

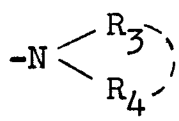

und B die Gruppe

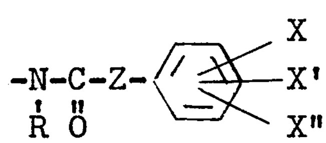

bedeuten;

- oder mit einem Amin der Formel (V)

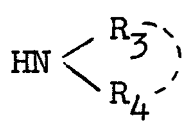

(V)

kondensiert, um zu einem Produkt der Formel (IX)

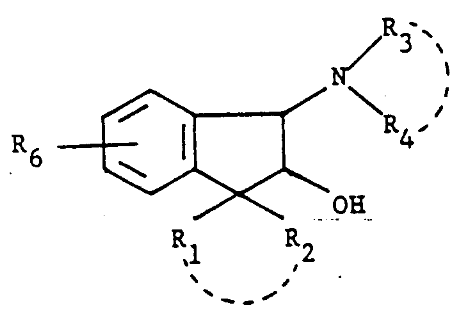

(IX)

zu gelangen, dessen Hydroxylfunktion man aktiviert und welches man mit einem Amin der Formel (X)

$NH_2R$ (X)

worin R wie vorstehend definiert ist, behandelt, um zu einem Produkt der Formel (XI)

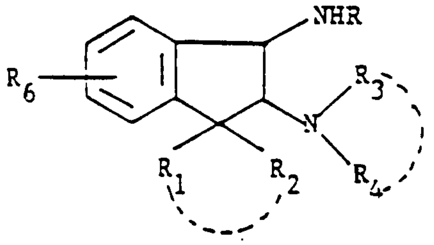

(XI)

zu gelangen, welches man mit einer Säure der Formel (VIII) oder einem funktionellen Derivat dieser Säure

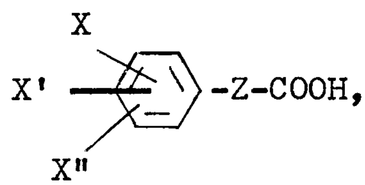

worin Z, X, X' und X" die vorstehenden Bedeutungen besitzen, kondensiert, um zu einem Produkt der Formel (I), worin A die Gruppe

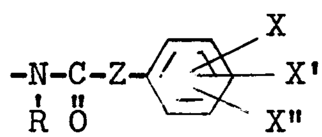

wiedergibt und B für die Gruppe

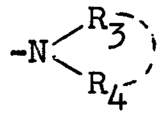

steht, zu gelangen, wobei die Verbindungen der Formel (I) aufgetrennt werden können, um die optisch aktiven Formen zu erhalten, welches Produkt man gewünschtenfalls mit einer Mineral- oder organischen Säure behandelt, um ein Salz zu erhalten.

**9.** Als Arzneimittel die Produkte der Formel (I), wie in einem der Ansprüche 1 bis 6 definiert, sowie deren Additionssalze mit pharmazeutisch verträglichen Säuren.

**10.** Als Arzneimittel die Produkte gemäß Anspruch 7 sowie deren Additionssalze mit pharmazeutisch verträglichen Säuren.

**11.** Pharmazeutische Zusammensetzungen, enthaltend als Wirkstoff zumindest eines der Arzneimittel, wie durch Anspruch 9 oder 10 definiert.

**12.** Als Zwischenprodukte die Produkte der Formeln (IV), (VI), (VII), (IX), (XI), worin $R_6$ kein Wasserstoff-

atom bedeutet und $R_1$ und $R_2$ kein Wasserstoffatom und keinen Alkylrest mit 1 bis 5 Kohlenstoffatomen bedeuten.